# EUROPEAN PATENT APPLICATION

(11) **EP 3 973 989 A1**
(43) Date of publication of application: **30.03.2022**
(21) Application number: 21199121.1
(22) Date of filing: 08.11.2018
(51) Int. Cl.: A61K 41/00, A61K 47/65, A61K 47/64

(54) **EXOSOMES COMPRISING RNA THERAPEUTICS**

(30) Priority: 08.11.2017 GB 201718471
(62) Divisional of application: 18806990.0
(71) Applicant: EVOX THERAPEUTICS LIMITED, Oxford OX4 4HG (GB)
(72) Inventor: ERRICHELLI, Lorenzo, Oxford, OX1 4LZ (GB); GUPTA, Dhanu, 14151 Huddinge (SE); HEAN, Justin, Oxford, OX2 7SS (GB); NORDIN, Joel, 11762 Stockholm (SE)
(74) Representative: Cooley (UK) LLP

(57) **Abstract**

The present invention pertains to extracellular vesicle (EV) therapeutics, wherein the EVs comprise nucleic acid (NA)-based therapeutics such as m RNAs, circular RNAs, mi RNAs, sh RNAs, circular RNA and/or DNA molecules. The NA therapeutics are loaded into EVs using inventive engineering protein and NA engineering strategies to enhance loading into EVs and to facilitate release of the NA cargo molecules inside target cells.

## Description

### Technical field

The present invention pertains to extracellular vesicle (EV) therapeutics, wherein the EVs comprise endogenously loaded RNA therapeutics such as mRNAs, circular RNAs, miRNAs, shRNAs and various other RNA therapeutics agents.

### Background to the invention

Nucleic acid-based therapeutics are approaching clinical utility at a rapid pace. Gene therapies, mRNA-based therapies, short oligonucleotide- and siRNA-based therapeutics are just some examples within the plethora of modalities within the RNA therapeutics landscape. As naked nucleic acids, typically RNA, are difficult to deliver *in vivo due* to rapid clearance, nuclease activity, lack of organ-specific distribution, and low efficacy of cellular uptake, specialized delivery vehicles are usually obligatory as a means of achieving bioactive delivery. This is especially the case for non-hepatic targets and for high-molecular weight RNA therapeutics such as mRNA and gene therapy.

Extracellular vesicles (such as exosomes) are typically nanometer-sized vesicles produced by most cell types and functioning as the body's natural transport system for proteins, nucleic acids, peptides, lipids, and various other molecules between cells. RNA-containing EVs have a number of potential therapeutic uses and are already being investigated as delivery vehicles for gene therapy, mRNA delivery, and delivery of short nucleic acids in various settings. WO2010/119256 represents the foundational invention in the field of nucleic acid delivery using exosomes and said application teaches the utility of exosomes for delivery of several types of nucleic acid cargo. The non-published application PCT/GB2017/051479 teaches improved methods for endogenous loading of various types of RNA therapeutics with the aid of RNA-binding proteins, which drag RNA of interest into EVs forming within parental cells. US14/502,494 represents another method for utilizing RNA-binding proteins to load certain RNA species, utilizing the so called The Targeted And Modular Exosome Loading (TAMEL) system.

However, despite these advances, there is still significant room in the art for improved loading of large and small RNA cargos into EVs in a specific and efficient manner. Furthermore, the actual bioactive delivery into target cells is a key aspect of any delivery system and the prior art has room for improvement also in this regard.

The TAMEL loading system described in US14/502,494 and the corresponding literature reference (Hung and Leonard, Journal of Extracellular Vesicles 2016, 5: 31027) has a number of disadvantages which the present invention attempts to overcome. The major disadvantage of the TAMEL system is that it is unable to achieve functional delivery of mRNA, i.e. the mRNA loaded into the exosomes by the TAMEL system is not translated by the cells when cells are exposed to these exosomes. The present application achieves bioactive delivery of the cargo mRNA.

Tutucci et al (An improved MS2 system for accurate reporting of the mRNA life cycle. Nat Methods. 2018 Jan; 15(1): 81―89) discusses the use of the MS2 protein for studying RNA localization and lifecycle and indicates that the MS2 protein as used by the TAMEL system binds with very high affinity to the RNA. This tight binding is reported by this paper to be problematic for the study of mRNA regulation. We surmise that in the context of EV loading this high affinity binding is again problematic because the tight binding of MS2 means that any RNA/nucleic acid that is loaded will not be released. Preventing release of mRNA will prevent normal translation and this would explain why no translation of the mRNA is observed in US14/502,494.

The TAMEL system does not appear to load all exosomes with nucleic acids and those that are loaded are loaded with very small numbers of nucleic acids (disadvantages of this variable and low level of loading are discussed in detail below). These variable and low levels of loading combined with the fact that what little nucleic acid that is loaded is then unlikely to be released and therefore not bioactive means that the TAMEL system has many disadvantages. The TAMEL system is not suitable for loading and delivery of clinically relevant quantities of bioactive nucleic acids. The present invention overcomes these significant disadvantages.

Another disadvantage of the TAMEL system is that it employs bacteriophage proteins which may elicit unwanted immunological responses when the EVs produced are delivered to patients. The present invention overcomes this disadvantage too.

### Summary of the invention

It is hence an object of the present invention to overcome the above-identified problems associated with loading and subsequent EV-mediated delivery of nucleic acid (NA)-based therapeutics into target cells, and to satisfy the existing needs within the art, for instance to enable reaching the right intracellular compartment with an NA therapeutics agent in question.

The present invention achieves this by utilizing novel EV engineering technology to load and release NA cargo. This is achieved by advanced engineering of polypeptide and polynucleotide construct to ensure not only highly efficient loading into EVs but also effective release of the NA in question. In a first aspect, this is achieved by providing an extracellular vesicle (EV) comprising at least one fusion polypeptide comprising at least one nucleic acid (NA)-binding domain and at least one exosomal polypeptide, wherein at least one NA-binding domain is one or more of Pumilio and FBF homology protein (PUF), Cas6, and/or an NA aptamer-binding domain, or any derivatives and/or domains and/or analogues thereof. The NA-binding domain may advantageously be present in several copies, and each NA cargo molecule may also be present in multiple copies with each and every copy having a plurality of binding sites for the NA-binding domain. Importantly, the NA-binding domains which form part of the fusion polypeptide and which are responsible for the interaction with the NA cargo molecule are releasable NA-binding domains, meaning that their binding of the NA cargo molecule is a reversible, releasable interaction. The releasable nature of the binding between the NA-binding domain and the NA cargo molecule is particularly advantageous as the present inventors have realized that overexpression of the NA cargo molecule in EV-producing cells allows for sufficiently high local concentrations to enable interaction between the NA-binding domain and the NA cargo molecule, while the lower concentration of the NA binding molecule in the target location (such as inside a target cell) allows for efficient release of the NA molecule, enabling its bioactive delivery.

The present invention also relates to the inventive fusion polypeptide constructs *per* se, and also polynucleotide constructs encoding for such polypeptides. Furthermore, the present invention relates to polynucleotide constructs encoding for the polypeptide constructs in question, as well as NA cargo polynucleotides which are engineered for stability and enhanced potency.

In additional aspects, the present invention relates to populations of EVs comprising the fusion polypeptide constructs and NA cargo molecules, as well as pharmaceutical compositions of EVs, EV populations, polynucleotide and/or polypeptide constructs, and/or cells and/or other vectors (such as viruses like adenoviruses, AAVs, lentiviruses, HSV, RSV, etc.) containing any of the above biomacromolecules.

In yet another aspect, the present invention relates to methods for producing EVs as per the invention. Such methods may comprise at least the steps of (i) introducing into an EV-producing cell at least one suitable polynucleotide construct, (ii) expressing in the EV-producing cell at least one polypeptide construct encoded for by the at least one polynucleotide construct, and (iii) collecting from the EV-producing cell EVs comprising the polypeptide obtainable via step (ii). Further, the present invention also relates to an *in vitro* method for intracellular delivery of at least one RNA cargo molecule, wherein said method comprises contacting a target cell (typically a target cell population) with at least one EV and/or at least one population of EVs.

In summary, the present invention provides for novel loading and release strategies for EV-mediated delivery of a multitude of NA therapeutics cargos. Advantageously, the present invention uses proteins which are all highly conserved amongst eukaryotes and are therefore unlikely to cause adverse immune responses when delivered to patients.

### Brief description of the figures

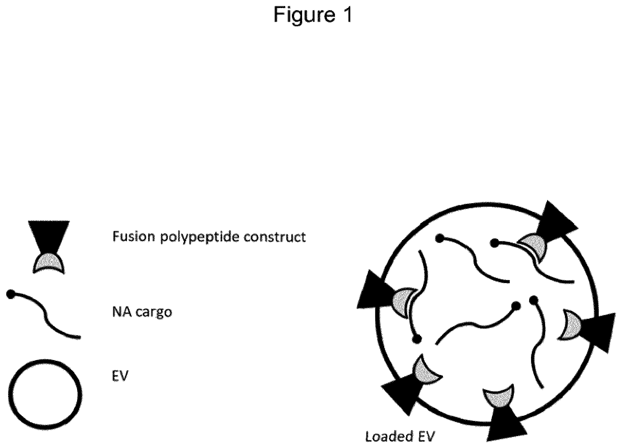

Figure 1: Schematic illustration of an EV loaded with an NA cargo molecule using the fusion polypeptide constructs as per the present invention.

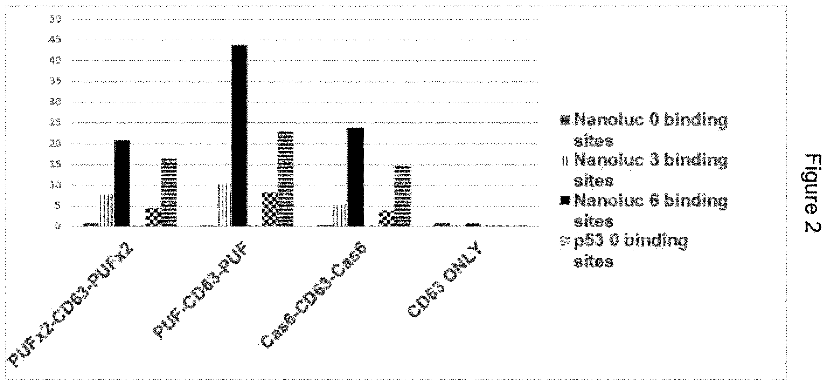

Figure 2: Graph showing loading, into MSC-derived EVs, of mRNA cargo molecules encoding NanoLuc (RTM) and p21, using fusion polypeptide constructs comprising CD63 as the exosomal polypeptide and PUF (in this case the NA-binding PUF domain is obtained from the human PUM1 protein) or Cas6 as the NA-binding domains. The experiment also included varying numbers of binding sites for the NA-binding domains, namely 0, 3 and 6 binding sites. Expression of only the exosomal polypeptide CD63 did not result in loading of any mRNA into the EVs (right). Expression of fusion polypeptides comprising PUF (left set of columns: two PUFs domain flanking CD63 both N terminally and C terminally, i.e. 4 PUF constructs in total) (second set of columns from left: one PUF domain flanking CD63 both N terminally and C terminally) and mutated Cas6 (second from right) did result in significant mRNA loading of both NanoLuc (RTM) and p21 mRNAs upon expression in the EV source cells. The loading of NanoLuc (RTM) was overall more efficient than the loading of p21, with up to around 45 copies of mRNA per EV.

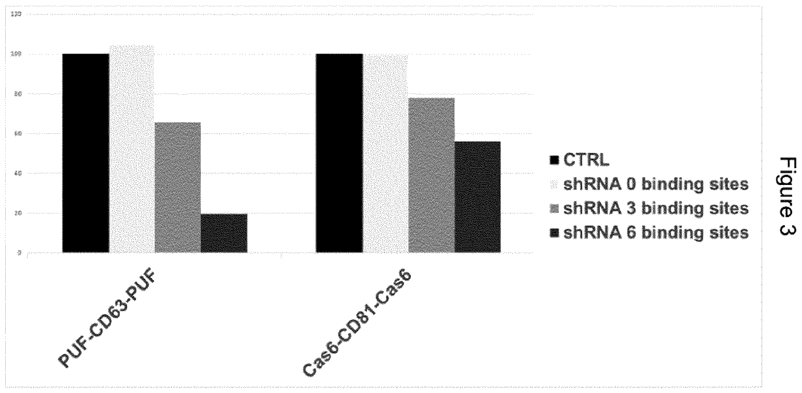

Figure 3: Silencing of gene expression *in vitro* in target cells upon EV-mediated delivery of an shRNA targeting huntingtin. Amnion cells were engineered to express the shRNA and fusion polypeptide constructs comprising as NA-binding domain either PUF (obtained from human PUM protein) or Cas6, combined with CD63 or CD81 as the EV polypeptides, respectively. The AE-EVs were collected from the cell culture and added to the target cells *in vitro.* The Y axis shows how the huntingtin expression level is decreased with increasing numbers of binding sites in the NA cargo molecule, resulting in approximately 80% knockdown using a fusion polypeptide comprising PUF-CD63-PUF to load the anti-huntingtin shRNA.

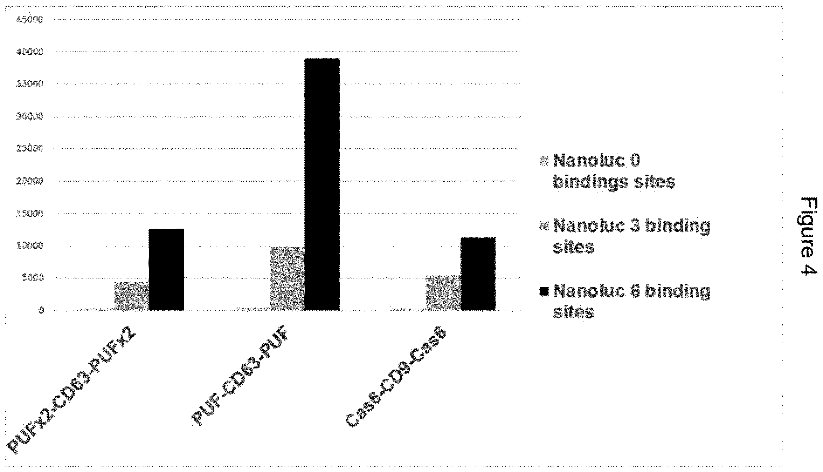

Figure 4: Expression of NanoLuc (RTM) as a reporter system in target Huh7 cells after HEK EV-mediated delivery of a NanoLuc (RTM) mRNA. The NanoLuc (RTM) mRNA cargo molecule was engineered to comprise 0, 3, or 6 binding sites for the NA-binding domains comprising the fusion polypeptide constructs, in this case PUFx2-CD63-PUFx2 (two PUF NA-binding polypeptides inserted both N terminally and C terminally of the exosomal polypeptide CD63), PUF-CD63-PUF, and Cas6-CD9-Cas6. The Y axis shows relative light (luminescence) units (RLU) normalized over µg of protein, indicating enhanced delivery and/or translation with increasing numbers of binding sites. Human PUM1 and also an NA-binding PUF obtained from Pumilio protein of D. melanogaster was evaluated in this assay.

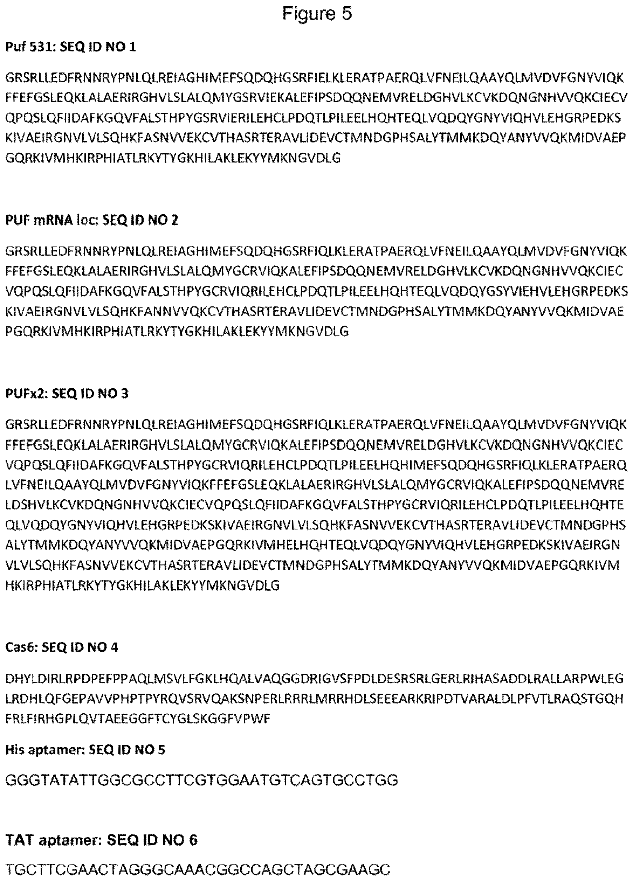

Figure 5: Sequences of SEQ ID No's 1 to 6.

### Detailed description of the invention

The present invention relates to improved loading, controlled release, and enhanced efficacy of EV-delivered NA therapeutics, using novel engineering approaches for introducing and delivering NA cargo in a bioactive fashion into target cells in vitro and/or in vivo.

For convenience and clarity, certain terms employed herein are collected and described below. Unless otherwise defined, all technical and scientific terms used herein have the same meaning as commonly understood by one of ordinary skill in the art to which this invention belongs.

Where features, aspects, embodiments, or alternatives of the present invention are described in terms of Markush groups, a person skilled in the art will recognize that the invention is also thereby described in terms of any individual member or subgroup of members of the Markush group. The person skilled in the art will further recognize that the invention is also thereby described in terms of any combination of individual members or subgroups of members of Markush groups. Additionally, it should be noted that embodiments and features described in connection with one of the aspects and/or embodiments of the present invention also apply mutatis mutandis to all the other aspects and/or embodiments of the invention. For example, the fusion polypeptides described herein in connection with the EVs are to be understood to be disclosed, relevant, and compatible with all other aspects, teachings and embodiments herein, for instance aspects and/or embodiments relating to the methods for producing or the EVs, or relating to the corresponding poly nucleotide constructs described herein. Furthermore, certain embodiments described in connection with certain aspects, for instance the administration routes of the EVs comprising the fusion polypeptides and optionally the NA drug cargo molecule, as described in relation to aspects pertaining to treating certain medical indications, may naturally also be relevant in connection with other aspects and/or embodiment such as those pertaining to the pharmaceutical compositions. Furthermore, all polypeptides and proteins identified herein can be freely combined in fusion proteins using conventional strategies for fusing polypeptides. As a non-limiting example, NA-binding domains (which are of polypeptide origins) described herein may be freely combined in any combination with one or more exosomal polypeptides, optionally combined with all other polypeptide domains, regions, sequences, peptides, groups herein, e.g. any multimerization domains, release domains, and/or targeting peptides. Also, exosomal polypeptides and/or NA-binding domains may be combined with each other to generate constructs comprising more than one exosomal polypeptide and/or more than one NA-binding domain. Moreover, any and all features (for instance any and all members of a Markush group) can be freely combined with any and all other features (for instance any and all members of any other Markush group), e.g. any NA-binding domain may be combined with any exosomal polypeptide. Furthermore, when teachings herein refer to EVs in singular and/or to EVs as discrete natural nanoparticle-like vesicles it should be understood that all such teachings are equally relevant for and applicable to a plurality of EVs and populations of EVs. As a general remark, the NA-binding domains, the exosomal polypeptides, the EV-producing cell sources, the additional domains and peptides, the NA cargo molecule, and all other aspects, embodiments, and alternatives in accordance with the present invention may be freely combined in any and all possible combinations without deviating from the scope and the gist of the invention. Furthermore, any polypeptide or polynucleotide or any polypeptide or polynucleotide sequences (amino acid sequences or nucleotide sequences, respectively) of the present invention may deviate considerably from the original polypeptides, polynucleotides and sequences as long as any given molecule retains the ability to carry out the desired technical effect associated therewith. As long as their biological properties are maintained the polypeptide and/or polynucleotide sequences according to the present application may deviate with as much as 50% (calculated using for instance BLAST or ClustalW) as compared to the native sequence, although a sequence identity that is as high as possible is preferable (for instance 60%, 70%, 80%, or e.g. 90% or higher). The combination (fusion) of e.g. several polypeptides implies that certain segments of the respective polypeptides may be replaced and/or modified and/or that the sequences may be interrupted by insertion of other amino acid stretches, meaning that the deviation from the native sequence may be considerable as long as the key properties (e.g. NA-binding, trafficking into EVs, targeting capabilities, etc.) are conserved. Similar reasoning thus naturally applies to the polynucleotide sequences encoding for such polypeptides. Any accession numbers or SEQ ID NOs mentioned herein in connection with peptides, polypeptides and proteins shall only be seen as examples and for information only, and all peptides, polypeptides and proteins shall be given their ordinary meaning as the skilled person would understand them. Thus, as above-mentioned, the skilled person will also understand that the present invention encompasses not merely the specific SEQ ID NOs and/or accession numbers referred to herein but also variants and derivatives thereof. All accession numbers referred to herein are UniProtKB accession numbers, and all proteins, polypeptides, peptides, nucleotides and polynucleotides mentioned herein are to be construed according to their conventional meaning as understood by a skilled person.

The terms "extracellular vesicle" or "EV" or "exosome" are used interchangeably herein and shall be understood to relate to any type of vesicle that is obtainable from a cell in any form, for instance a microvesicle (e.g. any vesicle shed from the plasma membrane of a cell), an exosome (e.g. any vesicle derived from the endo-lysosomal pathway), an apoptotic body (e.g. obtainable from apoptotic cells), a microparticle (which may be derived from e.g. platelets), an ectosome (derivable from e.g. neutrophils and monocytes in serum), prostatosome (e.g. obtainable from prostate cancer cells), or a cardiosome (e.g. derivable from cardiac cells), etc. The sizes of EVs may vary considerably but an EV typically has a nano-sized hydrodynamic diameter, i.e. a diameter below 1000 nm. Clearly, EVs may be derived from any cell type, in vivo, ex vivo, and in vitro. Preferred EVs include exosomes and microvesicles, but other EVs may also be advantageous in various circumstances. Furthermore, said terms shall also be understood to relate to extracellular vesicle mimics, cell membrane-based vesicles obtained through for instance membrane extrusion, sonication, or other techniques, etc. It will be clear to the skilled artisan that when describing medical and scientific uses and applications of the EVs, the present invention normally relates to a plurality of EVs, i.e. a population of EVs which may comprise thousands, millions, billions or even trillions of EVs. As can be seen from the experimental section below, EVs may be present in concentrations such as 10^{5,} 10⁸, 10¹⁰, 10¹¹, 10¹², 10¹³, 10¹⁴, 10¹⁵, 10¹⁸, 10²⁵, 10³⁰ EVs (often termed "particles") per unit of volume (for instance per ml), or any other number larger, smaller or anywhere in between. In the same vein, the term "population", which may e.g. relate to an EV comprising a certain fusion polypeptide between an exosomal polypeptide and an NA-binding domain which in turn may be binding an NA cargo molecule of interest, shall be understood to encompass a plurality of entities constituting such a population. In other words, individual EVs when present in a plurality constitute an EV population. Thus, naturally, the present invention pertains both to individual EVs and populations comprising EVs, as will be clear to the skilled person. The dosages of EVs when applied *in vivo* may naturally vary considerably depending on the disease to be treated, the administration route, the therapeutic activity, effects, and potency of the NA cargo molecule, any targeting moieties present on the EVs, the pharmaceutical formulation, etc. Furthermore, the EVs of the present invention may also comprise additional therapeutic agents, in addition to the NA cargo molecule. In some embodiments, the additional therapeutic agent may be at least one therapeutic small molecule drug. In some embodiments, the therapeutic small molecule drug may be selected from the group consisting of DNA damaging agents, agents that inhibit DNA synthesis, microtubule and tubulin binding agents, anti-metabolites, inducers of oxidative damage, anti-angiogenics, endocrine therapies, anti-estrogens, immuno-modulators such as Toll-like receptor agonists or antagonists, histone deacetylase inhibitors, inhibitors of signal transduction such as inhibitors of kinases, inhibitors of heat shock proteins, retinoids, inhibitors of growth factor receptors, anti-mitotic compounds, antiinflammatories, cell cycle regulators, transcription factor inhibitors, and apoptosis inducers, and any combination thereof. In further embodiments, the additional therapeutic agent may be an additional therapeutic nucleic acid-based agent. Such additional nucleic acid-based therapeutic agents may be selected from the group comprising single-stranded RNA or DNA, double-stranded RNA or DNA, oligonucleotides such as siRNA, splice-switching RNA, CRISPR guide strands, short hairpin RNA (shRNA), miRNA, antisense oligonucleotides, polynucleotides such as mRNA, plasmids, or any other RNA or DNA vector. Of particular interest are nucleic acid-based agents which are chemically synthesized and/or which comprise chemically modified nucleotides such as 2'-O-Me, 2'-O-Allyl, 2'-O-MOE, 2'-F, 2'-CE, 2'-EA 2'-FANA, LNA, CLNA, ENA, PNA, phosphorothioates, tricyclo-DNA, etc. In yet further embodiments, the EVs as per the present invention may comprise additional therapeutic agents which may be proteins and/or peptides. Such proteins and/or peptides may be present inside of the EVs, inserted into the EV membrane or in association with the EV membrane, or may be protruding from the EV into the extravesicular environment. Such therapeutic protein and/or peptide agents may be selected from a group of non-limiting examples including: antibodies, intrabodies, single chain variable fragments (scFv), affibodies, bi- and multispecific antibodies or binders, affibodies, darpins, receptors, ligands, enzymes for e.g. enzyme replacement therapy or gene editing, tumor suppressors (non-limiting examples include p53, p21, pVHL, APC, CD95, ST5, YPEL3, ST7, and/or ST15) viral or bacterial inhibitors, cell component proteins, DNA and/or RNA binding proteins, DNA repair inhibitors, nucleases, proteinases, integrases, transcription factors, growth factors, apoptosis inhibitors and inducers, toxins (for instance pseudomonas exotoxins), structural proteins, neurotrophic factors such as NT3/4, brain-derived neurotrophic factor (BDNF) and nerve growth factor (NGF) and its individual subunits such as the 2.5S beta subunit, ion channels, membrane transporters, proteostasis factors, proteins involved in cellular signaling, translation- and transcription related proteins, nucleotide binding proteins, protein binding proteins, lipid binding proteins, glycosaminoglycans (GAGs) and GAG-binding proteins, metabolic proteins, cellular stress regulating proteins, inflammation and immune system regulating proteins, mitochondrial proteins, and heat shock proteins, etc.

The terms "exosomal polypeptide", "exosomal protein", "exosomal carrier protein", "EV protein" and "EV polypeptide" and are used interchangeably herein and shall be understood to relate to any polypeptide that can be utilized to transport a polypeptide construct (which typically comprises, in addition to the exosomal polypeptide, an NA-binding domain, e.g. a polypeptide comprising an NA-binding domain) to a suitable vesicular structure, i.e. to a suitable EV. More specifically, these terms shall be understood as comprising any polypeptide that enables transporting, trafficking or shuttling of a fusion protein construct to a vesicular structure, such as an EV. Examples of such exosomal polypeptides are for instance CD9, CD53, CD63, CD81, CD54, CD50, FLOT1, FLOT2, CD49d, CD71 (also known as the transferrin receptor) and its endosomal sorting domain, i.e. the transferrin receptor endosomal sorting domain, CD133 , CD138 (syndecan-1), CD235a, ALIX, AARDC1, Syntenin-1, Syntenin-2, Lamp2b, syndecan-2, syndecan-3, syndecan-4, TSPAN8, TSPAN14, CD37, CD82, CD151, CD231, CD102, NOTCH1, NOTCH2, NOTCH3, NOTCH4, DLL1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, Fc receptors, interleukin receptors such as TNFR and gp130, immunoglobulins, MHC-I or MHC-II components, CD2, CD3 epsilon, CD3 zeta, CD13, CD18, CD19, CD30, TSG101, CD34, CD36, CD40, CD40L, CD44, CD45, CD45RA, CD47, CD86, CD110, CD111, CD115, CD117, CD125, CD135, CD184, CD200, CD279, CD273, CD274, CD362, COL6A1, AGRN, EGFR, GAPDH, GLUR2, GLUR3, HLA-DM, HSPG2, L1CAM, LAMB1, LAMC1, LFA-1, LGALS3BP, Mac-1 alpha, Mac-1 beta, MFGE8, SLIT2, STX3, TCRA, TCRB, TCRD, TCRG, VTI1A, VTI1B, other exosomal polypeptides, and any combinations or derivatives thereof, but numerous other polypeptides capable of transporting a polypeptide construct to an EV are comprised within the scope of the present invention. In several embodiments of the present invention, at least one exosomal polypeptide is fused to NA-binding domain, in order to form a fusion protein present in an EV for aiding the loading of the NA cargo molecule. Such fusion proteins may also comprise various other components to optimize their function(s), including linkers, transmembrane domains, cytosolic domains, multimerization domains, etc.

The terms "NA-binding domain" or "NA-binding polypeptide" or "NA-binding protein" are used interchangeably herein and relate to any domain that is capable of binding to a stretch of nucleotides. The NA-binding domains may bind to RNA, DNA, mixmers of RNA and DNA, particular types of NAs such as shRNA, miRNA, mRNA, gRNA, pri-miRNA, pre-miRNA, circular RNA, piRNA, tRNA, rRNA, snRNA, IncRNA, ribozymes, mini-circle DNA, plasmid DNA, etc. Furthermore, the NA-binding domain(s) may also bind to chemically modified nucleotides such as 2'-O-Me, 2'-O-Allyl, 2'-O-MOE, 2'-F, 2'-CE, 2'-EA 2'-FANA, LNA, CLNA, ENA, PNA, phosphorothioates, tricyclo-DNA, etc. Furthermore, the NA-binding domains may also bind to either particular sequences of NAs, to domains such as repeats, or to NA motifs, such as stem loops or hairpins. Such binding sites for the NA-binding domains may be naturally occurring in the NA cargo molecule of interest and/or may be engineered into the NA cargo molecule to further enhance EV loading and bioactive delivery. The binding affinity of the NA-binding domain for the nucleic acid is such that the nucleic acid is bound with high enough affinity to be shuttled into the exosomes but the affinity of binding is not so high as to prevent the subsequent release of the nucleic acid into the target cell such that the nucleic acid is bioactive once delivered to the target cell. Thus, importantly and in complete contrast to the prior art, the present invention relates to EVs loaded with NA cargo molecules with the aid of releasable NA-binding domains, wherein said NA-binding domains form part of fusion polypeptides with exosomal polypeptides. The NA-binding domains of the present invention have been selected to allow for programmable, modifiable affinity between the NA-binding domain and the NA cargo molecule, enabling production of EVs comprising fusion polypeptides comprising the NA-binding domain and at least one NA cargo molecule, wherein the NA-binding domain of the fusion polypeptide construct interacts in a programmable, reversible, modifiable fashion with the NA cargo molecule, allowing for both loading into EVs and release of the NA cargo molecule either in EVs and/or in or in connection with target cells. This is in complete contrast to the prior art, which merely allows for loading of mRNA molecules into exosomes using the MS2 protein, but wherein the MS2 protein remains bound to the mRNA, inhibiting its release and subsequent translation.

The present invention primarily relates to three groups of NA-binding domains, namely PUF proteins, CRISPR-associated polypeptides (Cas) and specifically Cas6 and Cas13, and various types of NA-binding aptamers. The present invention uses the term PUF proteins to encompass all related proteins and domains of such proteins (which may also be termed PUM proteins), for instance human Pumilio homolog 1 (PUM1), PUMx2 or PUFx2 which are duplicates of PUM1, etc., or any NA-binding domains obtainable from any PUF (PUM) proteins. PUF proteins are typically characterized by the presence of eight consecutive PUF repeats, each of approximately 40 amino acids, often flanked by two related sequences, Csp1 and Csp2. Each repeat has a 'core consensus' containing aromatic and basic residues. The entire cluster of PUF repeats is required for RNA binding. Remarkably, this same region also interacts with protein co-regulators, and is sufficient to rescue, to a large extent, the defects of a PUF protein mutant, which makes the PUF proteins highly suitable for mutations used in the present invention. Furthermore, PUF proteins are highly preferred examples of releasable NA-binding domains which bind with suitable affinity to NA cargo molecules, thereby enabling a releasable, reversible attachment of the PUF protein to the NA cargo. PUF proteins are found in most eukaryotes and is involved in embryogenesis and development. PUFs has one domain that binds RNA that is composed of 8 repeats generally containing 36 amino acids, which is the domain typically utilized for RNA binding in this patent application. Each repeat binds a specific nucleotide and it is commonly the amino acid in position 12 and 16 that confer the specificity with a stacking interaction from amino acid 13. The naturally occurring PUFs can bind the nucleotides adenosine, uracil and guanosine, and engineered PUFs can also bind the nucleotide cytosine. Hence the system is modular and the 8-nucleotide sequence that the PUF domain binds to can be changed by switching the binding specificity of the repeat domains. Hence, the PUF proteins as per the present invention can be natural or engineered to bind anywhere in an RNA molecule, or alternatively one can choose PUF proteins with different binding affinities for different sequences and engineer the RNA molecule to contain said sequence. There is furthermore engineered and/or duplicated PUF domains that bind 16-nucleotides in a sequence-specific manner, which can also be utilized to increase the specificity for the NA cargo molecule further. Hence the PUF domain can be modified to bind any sequence, with different affinity and sequence length, which make the system highly modular and adaptable for any RNA cargo molecule as per the present invention. PUF proteins and regions and derivatives thereof that may be used as NA-binding domains as per the present invention include the following non-limiting list of PUF proteins: FBF, FBF/PUF-8/PUF-6,-7,-10, all from C. elegans; Pumilio from D. melanogaster; Puf5p/Mpt5p/Uth4p, Puf4p/Ygl014wp/Ygl023p, Puf5p/Mpt5p/Uth4p, Puf5p/Mpt5p/Uth4p, Puf3p, all from S. cerevisiae; PufA from Dictyostelium; human PUM1 (Pumilio 1, sometimes known also as PUF-8R) and any domains thereof, polypeptides comprising NA-binding domains from at least two PUM1, any truncated or modified or engineered PUF proteins, such as for instance PUF-6R, PUF-9R, PUF-10R, PUF-12R, and PUF-16R or derivatives thereof; and X-Puf1 from Xenopus. Particularly suitable NA-binding PUFs as per the present invention includes the following: PUF 531 (as a non-limiting example illustrated by SEQ ID NO 1), PUF mRNA loc (sometimes termed PUFengineered or PUFeng) (as a non-limiting example illustrated by SEQ ID NO 2), and/or PUFx2 (as a non-limiting example illustrated by SEQ ID NO 3), and any derivatives, domains, and/or regions thereof. The PUF/PUM proteins are highly advantageous as they may be selected to be of human origin. Sequences of SEQ ID NO's 1 to 3 are depicted in Figure 5.

Proteins of human origin, rather than those of bacteriophage origin such as the MS2 protein used in US14/502,494, are beneficial because they are less likely to illicit an adverse immune response. Furthermore, MS2 interacts with a stem loop of bacteriophage origin, which unlike the PUF proteins imply that a prokaryotic NA sequence and motif need to be introduced into the NA molecule of choice. Clearly, this insertion of a stem loop structure of bacteriophage origin and structure may interfere with mRNA translation, resulting in non-functional mRNA cargo molecules, or even trigger immunotoxicity.

Thus, in advantageous embodiments, the present invention relates to eukaryotic NA-binding proteins fused to exosomal proteins. In a preferred embodiment, the NA-binding domain(s) is(are) from the PUF family of proteins, for instance PUF531, PUFengineered, and/or PUFx2. Importantly, PUF proteins are preferably used in the EV-mediated delivery of mRNA or shRNA, which due to the sequence-specificity of the PUF proteins enables highly controlled and specific loading of the NA drug cargo. In preferred embodiments, the PUF protein(s) are advantageously combined with either transmembrane or soluble exosomal proteins. Advantageous fusion protein constructs include the following non-limiting examples: CD63-PUF531, CD63-PUFx2, CD63-PUFengineered, CD81-PUF531, CD81-PUFx2, CD81-PUFengineered, CD9-PUF531, CD9-PUx2, CD9-PUFengineered, and other transmembrane-based fusion proteins, preferably based on tetraspanin exosomal proteins fused to one, two or more PUF proteins. Advantageous fusion proteins comprising PUF proteins and at least one soluble exosomal protein include the following non-limiting examples: syntenin-PUF531, syntenin-PUx2, syntenin-PUFengineered, syndecan-PUF531, syndecan-PUx2, syndecan-PUFengineered, Alix-PUF531, Alix-PUx2, Alix-PUFengineered, as well as any other soluble exosome protein fused to a PUF protein.

The fact that the PUF proteins have modifiable sequence-specificity for the target NA cargo molecule makes them ideal NA-binding domains for fusing to exosomal polypeptide partner(s). Thus, in preferred embodiments of the present invention, the EVs are loaded with NA cargo molecules using releasable NA-binding domains (as part of fusion proteins with exosomal proteins), wherein the interaction between the NA-binding domain and the NA cargo molecule is advantageously based on specificity for a target nucleotide sequence and not based on a target nucleotide secondary structure (as secondary structures do not enable sequence specificity). In preferred embodiments, the NA cargo molecule is engineered to comprise and/or naturally comprises the target nucleotide sequence for the PUF protein chosen as the NA-binding domain. Such target nucleotide sequences may as abovementioned, for example, be part of the 3'UTR of an mRNA or may be introduced into any NA cargo molecule such as an mRNA, shRNA, miRNA, IncRNA, DNA, etc., allowing for the PUF protein to bind to the NA cargo molecule. The PUF binding site on the NA cargo molecule is typically longer than the sequence bound by many other RNA-binding proteins, such as MS2 which merely recognizes 4 nucleotides and a stem loop in combination, so the preferred stretch of nucleotides on the target binding site may be for instance 5 nucleotides (nt), 6 nt, 7 nt, 8 nt, 9 nt, 10 nt, 11 nt, 12 nt, 13 nt, 14 nt, 15 nt, 16 nt, 17 nt, 18 nt, 19 nt, or even 20 nt and longer, depending on the need for modifiable sequence specificity of the NA-binding domain. In a preferred embodiment, the PUF protein is specific for a natural and/or artificially occurring NA cargo molecule binding site which is 6 nt, 8 nt, 9 nt, 10 nt, 12 nt, or 16 nt in length.

CRISPR-associated polypeptides (Cas) represent another group of NA-binding domains, and may include in particular Cas6 (as a non-limiting example illustrated by SEQ ID NO 4, the sequence of which is depicted in Figure 5) and Cas13 as well as any other RNA binding Cas molecule. Cas6 binds precursor CRISPR RNA (crRNA) with high affinity and processes it for later incorporation into for example Cas9. The cleavage rate of the RNA molecule can be modulated and highly defined, hence the association time between the RNA molecule and Cas6 can also be defined in a very accurate fashion, which is important for the purposes of the present invention. Mutant versions of Cas6 or Cas13 may be used which have been mutated to increase or decrease efficiency of RNA cleavage. Mutant versions of Cas6 or Cas13 may be used which have been mutated to increase or decrease the affinity of RNA binding. This will be an advantage for instance when the RNA cargo molecule is to be released in the recipient cell. The defined association time can then be modulated to release the RNA molecule inside the vesicles, but not in the producer cell. The RNA sequence that Cas6 can recognize can be engineered to be inserted into an NA molecule of interest. Cas13 can be engineered to only bind its defined RNA target and not degrade it. By changing the sequence of the sgRNA molecule the Cas13-sgRNA complex can be modulated to bind any RNA sequence between 20-30 nucleotides. For instance, the use of NA-binding domains from Cas proteins is especially advantageous for the delivery of short RNAs, for instance shRNAs or miRNAs, In such instances the cleavage activity of the selected Cas polypeptides may be used to release e.g. an shRNA cargo molecule from a binding site to which e.g. Cas6 has bound. Furthermore, as is the case with the PUF proteins, Cas proteins are highly preferred examples of releasable NA-binding domains which bind with suitable affinity to NA cargo molecules, thereby enabling a releasable, reversible attachment of the Cas protein to the NA cargo. As with the PUF-based NA-binding domains, the Cas proteins represent a releasable, irreversible NA-binding domain with programmable, modifiable sequence specificity for the target NA cargo molecule, enabling higher specificity at a lower total affinity, thereby allowing for both loading of the NA cargo into EVs and release of the NA cargo in a target location.

NA aptamer-binding domains are another group of NA-binding domains as per the present invention. Such NA aptamer-binding domains are domains, regions, stretches of amino acids, or entire polypeptides or proteins that can be bound with specificity by NA-based aptamers. Aptamers are RNA sequences that form secondary and/or tertiary structures to recognize molecules, similar to the affinity of an antibody for its target antigen. Hence these RNA molecules can recognize specific amino acid sequences with high affinity. RNA aptamers are applied in the present invention by inserting particular nucleotide sequences into the NA molecule to recognize specific amino acid sequences. Such amino acid sequences can be engineered into and/or next to the exosomal carrier polypeptide to enable the aptamer (which is engineered into and/or next to the NA cargo molecule) to bind to it, thereby shuttling the NA cargo molecule into EVs with the aid of the exosomal polypeptide. Two aptamers with suitable characteristics are a His-aptamer (as a non-limiting example illustrated by SEQ ID NO 5) with high affinity for a stretch of histidine (His) amino acids and an aptamer towards the HIV Tat domain (as a non-limiting example illustrated by SEQ ID NO 6). The aptamer sequence(s) are preferably inserted in the 3' and/or 5' untranslated region of an mRNA or unspecific region of non-coding RNAs. Two or more aptamers can also be combined into one NA cargo molecule to increase the specificity and avidity to the exosomal carrier protein. Sequences of SEQ ID NO's 5 and 6 are depicted in Figure 5. Importantly, all the NA-binding domains of the present invention provide for programmable, sequence-specific, reversible, releasable binding to the NA cargo molecule, for instance mRNA, shRNA, or miRNA, which is in complete contrast to the high-affinity, irreversible binding to RNA found in the prior art. In preferred embodiments of the present invention, the NA-binding domains are either PUF proteins or Cas proteins, due to their easily programmable nature and sequence specificity combined with their reversible, releasable binding to NA cargo molecules. Importantly, the sequence specificity of Cas proteins and PUF proteins as NA-binding domains is preferably based on interaction with at least 6 nt, preferably at least 8 nt on the target NA molecule, which when combined with a low-affinity interaction allows for high productive EV-mediated delivery of the NA cargo molecule. The at least 6 nt binding site on the NA cargo molecule is preferably present in a contiguous sequence of nucleotides. The binding site of the NA cargo molecule thus preferably corresponds in length to two codons.

In a first aspect, the present invention relates to extracellular vesicles (EVs) comprising at least one fusion polypeptide comprising at least one nucleic acid (NA)-binding domain and at least one exosomal polypeptide, wherein the at least one NA-binding domain may be one or more of PUF, a CRISPR-associated (Cas) polypeptide, and/or an NA aptamer-binding domain. As a result of presence of the NA-binding domain, the EVs typically further comprise at least one NA cargo molecule. Normally, the number of NA cargo molecules that are comprised in each and every EV is considerable, which is a clear improvement over the prior art which normally achieves a very low loading efficacy. In the case of the present invention, the inventive design of the fusion polypeptide constructs means that the at least one NA cargo molecule is very efficiently transported into the EV (with the help of the fusion polypeptide) followed by a significantly improved release process. The releasable nature of the binding between the NA-binding domain (which is comprised in the fusion polypeptide) and the NA cargo molecule is a key aspect of the present invention, as it allows for binding of NA cargo molecules in the EV-producing cells (where NA cargo molecules are normally overexpressed) while enabling delivery of bioactive NA molecules in and/or near the target cell.

Thus, unlike in the prior art, a programmable, lower affinity interaction between the NA-binding domain and the NA cargo molecules enables the present invention to efficiently load EVs in EV-producing cells, whilst also enabling release of NA cargo in suitable locations (typically inside a target cell) where the lower affinity and the releasable nature of the interaction between the NA cargo molecule and the NA-binding domain is highly advantageous. Furthermore, unlike the prior art which merely discloses MS2 as a high-affinity RNA-binding protein binding to 4 nts and a stem loop, the present invention allows for sequence-specific low-affinity or medium-affinity binding to stretches of nucleotides that are longer and thereby more specific, for instance 6 nt in length, or 8 nt in length.

The longer length of binding site enables a range of different mutations to be introduced which generate binding sites with a range of modified binding affinities, thus producing the programmable lower affinity interactions mentioned above. For instance, introduction of a single point mutation into a 6 or 8 nucleotide region will subtly modify the binding affinity, whereas, even a single mutation in the shorter 4 nucleotide binding region of MS2 is known to significantly affect the binding affinity of MS2 for the RNA. The longer length of nucleic acid provides more scope to introduce one or more mutations which affect the binding affinity of the protein for the nucleic acid. Similarly, requiring a longer stretch of nucleotides to be bound results in a larger number of amino acids which are capable of interacting with the longer nucleotide sequence and thus providing more possibilities for mutating those interacting amino acids and again producing a larger range of possible protein mutants with a variety of binding affinities. Both the longer nucleotide binding site and the larger protein binding sites of PUF, Cas6 and Cas13 provide advantages in enabling a greater range of affinities to be achieved by mutation than could be achieved by mutation of the MS2 protein or the MS2 RNA sequence. Thus, this longer sequence affords greater possibilities to engineer the nucleic acid and/or the binding protein to tailor the binding affinity specifically to an individual cargo of interest if needed to improve the release of that cargo nucleic acid. As has been discussed above, the ability to control the affinity of binding to the nucleotide cargo and thus modify and control the releasability of the nucleotide cargo is a significant advantage of the present invention over the prior art resulting in delivery and release of bioactive nucleic acids.

In one embodiment, the NA cargo molecule may be selected from the group comprising shRNA, miRNA, mRNA, gRNA, pri-miRNA, pre-miRNA, circular RNA, piRNA, tRNA, rRNA, snRNA, IncRNA, ribozymes, mini-circle DNA, plasmid DNA, but essentially any type of NA molecule can be comprised in the EVs as per the present invention. Both single-stranded and double-stranded NA molecules are within the scope of the present invention, and the NA molecule may be naturally occurring (such as RNA or DNA) or may be a chemically synthesized RNA and/or DNA molecule which may comprise chemically modified nucleotides such as 2'-O-Me, 2'-O-Allyl, 2'-O-MOE, 2'-F, 2'-CE, 2'-EA 2'-FANA, LNA, CLNA, ENA, PNA, phosphorothioates, tricyclo-DNA, etc. Importantly, although the present invention is highly suitable for endogenous loading of NA cargo molecules (for instance mRNA, circular RNA, shRNA, etc.) it is also applicable to loading with exogenous NA molecules which may be loaded by exposing EV-producing cells to the NA molecule in question and/or by co-incubation or formulation with the EVs per se.

In a specific embodiment the NA-binding domain is Cas6 or Cas13 and the NA cargo molecule is an shRNA. The combination of Cas6/Cas13 with shRNA cargo is advantageous because the innate activity of Cas6/Cas13 will result in the cleavage of the shRNA from the NA-binding domain(s) to which Cas6 or Cas13 have bound, thereby releasing the shRNA from the complex between the fusion protein and the NA-binding domain. Additionally, because the shRNA does not require any cap or polyA tail to function once released into the cytoplasm the cleaved shRNA is immediately ready to silence genes with high activity.

In embodiments of the present invention, the NA cargo molecules as per the present invention comprise (i) at least one binding site for the NA-binding domain of the fusion polypeptide and (ii) a therapeutic polynucleotide domain. In preferred embodiments, the NA cargo molecules comprises at least two binding sites and even more preferably a higher number of binding sites, e.g. 3, 4, 5, 6, 7, 8, 9, 10, 15, or an even greater number. The inventors have realized that including 4-8 binding sites yields optimal loading of the NA cargo molecule into EVs without negatively impacting the release and bioactive delivery of the cargo. The binding sites for the NA-binding domain, can be genetically engineered into and/or flanking the 3' and/or 5' UTR and/or by sequence optimization be placed in the coding region of the NA cargo molecule.

The NA cargo molecules as per the present invention may advantageously comprise at least one cleavage site between the at least one binding site and the therapeutic polynucleotide domain, in order to enable release of the therapeutic part of the NA cargo molecule.

Generally, the NA cargo molecule may be intended to carry out a range of functions, for instance encode for a protein of interest (such as a protein with a therapeutic activity), silence a target nucleotide sequence via antisense interaction with the target, switch and/or block splicing, mediate cleavage of a target nucleotide sequence e.g. via RNase H-mediated cleavage or RISC complex mediated RNA interference (RNAi). In embodiments of particular interest, the NA cargo molecule may carry out more than one function, for instance it may encode for a protein of interest and comprise an NA sequence which may have e.g. a guiding function. A particularly advantageous example of this is an NA molecule encoding for a CRISPR-associated protein (such as Cas, Cas9 (in a non-limiting example Cas9 with accession number Q99ZW2 as shown in SEQ ID NO: 7), and/or Cas6) and also comprising a guide strand for directing the CRISPR-associated protein to a target sequence for gene editing and/or a correcting DNA strand for e.g. homology directed repair. In such embodiments, it is particularly advantages to include cleavage sites in the NA molecule, to enable release of either one or both of the protein encoded by the NA cargo molecule and the guide strand comprised in the NA cargo molecule.

Non-limiting examples of proteins that may be encoded for by the NA cargo molecule include the following: antibodies, intrabodies, single chain variable fragments, affibodies, enzymes, transporters, tumor suppressors (non-limiting examples include p53, p21, APC, CD95, ST5, YPEL3, ST7, and/or ST15), viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, nucleotide binding proteins, heat shock proteins, CRISPR-associated proteins, cytokines, cytokine receptors, caspases and any combination and/or derivatives thereof.

The designs of both the NA cargo molecule and the fusion polypeptide constructs are key to loading, release, and bioactive delivery, e.g. into target cells and/or into particular organs, tissues, and bodily compartments. The inventors have discovered that particularly advantageous embodiments are EVs comprising fusion polypeptides which comprises at least one exosomal polypeptide flanked on one or both sides by at least one NA-binding domain (i.e. at least one NA-binding domain either on one side or on each side). Alternatively, the NA-binding domain may in various instances by inserted into the exosomal polypeptide in at least one location (for instance on an extravesicular loop of e.g. CD63), for instance when it is desirable to display the NA-binding domain on the outside of the EV to enhance exogenous loading. The exosomal polypeptide may be flanked immediately C and/or N terminally, but the most advantageous design is to include a linker peptide between the exosomal polypeptide and the NA-binding domains, to provide spacing and flexibility for maintained activity of both the exosomal polypeptide(s) and the NA-binding domain(s). Such linkers may advantageously be glycine-serine (GS) linkers containing a particular number of repeats. The inventors have realized that either 1 to 4 repeats are the most advantageous, providing enough flexibility without rendering the fusion polypeptide too unstructured, however longer linkers and non-GS-based linkers are also within the scope of the present application. As above-mentioned, for applications involving exogenous loading of NA cargo molecules, EVs preferably comprise fusion polypeptides which comprises at least one exosomal polypeptide fused to at least one NA binding domain on its N terminal, and/or its C terminal and/or in any extravesicular (i.e. present outside of the EV) regions of the exosomal polypeptide, in order to expose the NA binding domain on the surface of exosome.

Design and selection of the exosomal polypeptide component of the fusion polypeptide construct is key to enable efficient EV formation, NA loading into the EVs, and also release of the NA cargo molecule. The exosomal proteins may be selected from the following non-limiting examples: CD9, CD53, CD63, CD81, CD54, CD50, FLOT1, FLOT2, CD49d, CD71, CD133, CD138, CD235a, ALIX, AARDC1, Syntenin-1, Syntenin-2, Lamp2b, TSPAN8, syndecan-1, syndecan-2, syndecan-3, syndecan-4, TSPAN14, CD37, CD82, CD151, CD231, CD102, NOTCH1, NOTCH2, NOTCH3, NOTCH4, DLL1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, TNFR, gp130, Fc receptors, interleukin receptors, immunoglobulins, MHC-I or MHC-II components, CD2, CD3 epsilon, CD3 zeta, CD13, CD18, CD19, CD30, CD34, CD36, CD40, CD40L, CD44, CD45, CD45RA, CD47, CD86, CD110, CD111, CD115, CD117, CD125, CD135, CD184, CD200, CD279, CD273, CD274, CD362, COL6A1, AGRN, EGFR, GAPDH, GLUR2, GLUR3, HLA-DM, HSPG2, L1CAM, LAMB1, LAMC1, LFA-1, LGALS3BP, Mac-1 alpha, Mac-1 beta, MFGE8, SLIT2, STX3, TCRA, TCRB, TCRD, TCRG, VTI1A, VTI1B, other exosomal polypeptides, and any combinations thereof.

As above-mentioned, the EVs as per the present are loaded with the NA cargo molecule with the aid of the fusion polypeptide. Without wishing to be bound by any theory, it is surmised that the loading takes place in connection with the formation of the EV inside the EV-producing cell or exogenously by incubating NA cargo molecule(s) with engineered EVs. The fusion polypeptide may normally bind to the NA cargo molecule (such as an mRNA molecule co-expressed in the EV-producing cell) and transport it into the vesicle which is then released as an EV. As mentioned, the NA cargo molecule may be expressed in the same EV-producing cell as the fusion polypeptide and/or it may be loaded exogenously into an EV once the EV is formed and optionally purified. Co-expression in the EV-producing cell of the NA cargo is a highly advantageous embodiment, as the EV production takes place in a single step in a single cell, which enables scaling the process and simplifies both upstream and downstream processing. The NA cargo molecule (e.g. an mRNA, an shRNA, a miRNA, a circular RNA, a DNA, an antisense oligonucleotide, etc.) may be expressed from the same polynucleotide constructs as the fusion polypeptide, or it may also be expressed from another construct. Both methods have advantages: the use of one construct ensure that both the fusion polypeptide and the NA cargo molecule are translated/transcribed together whereas the use of more than one construct enables differential expression of the two components, e.g. a higher expression level of either the fusion polypeptide or the NA cargo molecule. In preferred embodiments, the polynucleotide construct from which the fusion polypeptide and/or the NA cargo molecule are expressed is advantageously stably introduced into the EV-producing cells, to enable consistent, reproducible and high-yield production of the NA-loaded EVs. In a preferred embodiment, the EV-producing cells are stably transfected and/or transduced with bicistronic or multicistronic vectors (also known as constructs or polynucleotides, etc.) comprising the fusion polypeptide and the NA cargo molecule. Such bicistronic or multicistronic construct may comprise e.g. IRES element(s) or 2A peptide linkages, allowing for the expression of both (i) the fusion polypeptide comprising the NA-binding domain and the exosomal protein, and (ii) the NA cargo molecule of interest, for instance an mRNA, an shRNA, a miRNA, or any other type of NA cargo molecule. In addition to using bicistronic or multicistronic vectors, multiple or bidirectional promoters represent another tractable method for stably inserting a single construct encoding for the two components of interest that are to be loaded into the EVs according to the present invention. Clearly, in alternative embodiments, two or more constructs (for instance plasmids) may also be transfected and/or transduced into EV-producing cells, although the use of single constructs may be advantageous as it may enable equimolar concentrations of the fusion polypeptide (and thus the NA-binding domain) and the NA cargo molecule *per se.* Importantly, the EV-producing cells of the present invention are normally designed to overexpress the at least one polynucleotide construct, which allows for appropriate production of the NA cargo molecule at a suitable concentration in the EV-producing cell, thereby allowing for the reversible, releasable attachment of the NA-binding domain to the NA molecule. Overexpression of the polynucleotide(s) is an important tool that allows for creating a relatively high NA cargo molecule concentration in the EV-producing cell, while allowing at the same time for release of the NA cargo molecule in the target cell where the concentration of the NA cargo molecule is lower. This is especially relevant for the PUF and Cas proteins.

In a further embodiment, the EVs as per the present invention may comprise at least one targeting moiety, to enable targeted delivery to a cell, tissue, organ, and/or compartment of interest. The targeting moiety may be comprised in the fusion polypeptide itself, which is especially advantageous when using an exosomal polypeptide with a transmembrane domain to enable display of the targeting moiety on the surface of the EVs. Targeting moieties may be proteins, peptides, single chain fragments or any other derivatives of antibodies, etc. The targeting moiety may also form part of a separate polypeptide construct which is comprised in the EV. Further, the fusion polypeptides comprised in the EVs of the present invention may also comprise various additional moieties to enhance the bioactive delivery. Such moieties and/or domains may include the following non-limiting examples of functional domains: (i) multimerization domains which dimerize, trimerize, or multimerize the fusion polypeptides to improve EV formation and/or loading, (ii) linkers, as above-mentioned, to avoid steric hindrance and provide flexibility, (iii) release domains, such as cis-cleaving elements like inteins, which have self-cleaving activity which is useful for release of particular parts of the fusion polypeptide and/or the NA cargo, (iv) RNA cleaving domains for improved release of the RNA in recipient cells, for instance domains encoding for nucleases such as Cas6, Cas13, (v) endosomal escape domains, such as HA2, VSVG, GALA, B18, etc., and/or (vi) nuclear localization signals (NLSs).

The present invention also relates to various inventive modifications of the NA cargo molecule, which are key to ensure high efficiency of loading, release and bioactive delivery. For instance, by designing the NA cargo molecule to be either linear or circular one can increase or decrease aspects such as loading efficiency and stability. Furthermore, by optimizing the design of the sequence it is also possible to influence secondary and tertiary structures of the NA cargo, which can further facilitate loading, by facilitating the easy accessibility of NA binding domain to the target NA.

In yet another advantageous embodiment, the NA cargo molecule may comprise additional moieties to increase potency, either by enhancing loading, improving release, increasing tissue-specific activity, and/or increase the stability of the NA cargo molecule. For instance, the NA cargo molecule may comprise one or more of the following: (i) a site for miRNA binding, wherein such site optionally is tissue and/or cell type specific, to drive preferential cell and/or tissue specific activity, (ii) at least one stabilizing domain, such as a long PolyA tail or more than one PolyA tail (for instance 2 or 3 or even 4 PolyA tails), (iii) at least one stem loop structure in the 5' and/or 3' UTR, in order to inhibit nuclease degradation, (iv) an RNA polymerase to drive transcription of the NA cargo molecule, (v) codon-optimized sequences to increase mRNA stability, (vi) at least one hybrid UTR in the 5' and/or 3' end to increase mRNA translation efficiency, and/or (vii) ribozyme(s).

As above-mentioned, EVs are typically present not as single vesicles but in a substantial plurality of vesicles, and the present invention hence also relates to populations of EVs. In advantageous embodiments, the average number of NA cargo molecules per EV throughout such a population is above on average one (1) NA cargo molecule per EV, preferably above 10 NA cargo molecule per EV, and even more preferably above 100 NA cargo molecule per EV. However, throughout the population there may also be EVs which do not comprise any NA cargo molecules, and the present invention may thus also relate to populations of EVs which comprise on average less than one (1) NA cargo molecule per EV.

Importantly, the prior art typically merely yields loading of the RNA cargo into a small fraction of the EVs. For instance, the TAMEL system described in US14/502,494 does not appear to enable quantifiable loading into EVs and specifically not into exosomes. This likely indicates that the TAMEL system results in zero to sub-single percentage loading of single EVs. The inventors of the TAMEL system reports that loading of an RNA molecule into exosomes is enhanced when using the TAMEL system at most 7-fold, whereas the present invention improves productive loading of e.g. mRNA and other NA cargo molecules by typically at least 10-fold, preferably at least 25-fold, but frequently by at least 50-fold, and preferably by at least 70-fold, as compared to (i) EVs without NA-binding domain present in the fusion protein and/or without binding site for the NA-binding domain in the NA cargo molecule, (ii) EVs without the fusion protein *per se* (for instance as shown in Figure 2) , (iii) un-engineered EVs which are only passively loaded with the NA cargo molecule, and/or (iv) a given internal NA control molecule. Thus, the present invention provides for a way of loading considerably more NA cargo molecules into a given population of EVs, and importantly the present invention also enables loading a significantly higher proportion of EVs as compared to the prior art. In one embodiment, the present invention relates to EV populations wherein at least 5%, at least 10%, at least 20%, at least 50%, at least 70%, at least 75%, at least 80%, at least 85%, at least 90%, and/or at least 95% of all EVs comprise an NA cargo molecule in question.

As abovementioned, a crucial difference between the present invention and US14/502,494 and other prior art documents relate to the ineffective and importantly uneven distribution of fusion polypeptides across whole populations of EVs. The MS2 protein used in US14/502,494, for instance, is only present in a small fraction of EVs, which results in unevenly distributed loading of mRNA cargo across EV populations. Conversely, the fusion proteins of the present invention are evenly distributed across entire EV populations, which means that essentially each and every EV comprises at least one fusion polypeptide as per the present invention and normally at least one NA cargo molecule. Thus, in one embodiment, the present invention relates to an EV composition comprising essentially two EV subpopulations, wherein (i) the first EV subpopulation comprises on average more than one fusion polypeptide (comprising the NA-binding domain and the exosomal polypeptide) per EV, and (ii) wherein the second EV subpopulation comprises the NA cargo molecule in question combined with on average more than one fusion polypeptide per EV. In contrast, the prior art, for instance US14/502,494, teaches EVs which comprise very few fusion polypeptides per EV, typically less than 1 fusion polypeptide per 10 EVs, which clearly implies that the productive loading and delivery of an NA cargo molecule that is dependent on said fusion protein will be significantly lower than is the case in the present application. Without wishing to be bound by any theory, it is surmised that the reason for the prior art's failure to achieve higher loading of the fusion protein into EVs results from the fact that MS2 and similar non-eukaryotic proteins do not shuttle efficiently into exosomes and/or that they trigger toxicity, two issues that are addressed by the present invention.

In another aspect, the present invention relates to inventive fusion polypeptides comprising at least one NA-binding domain and at least one exosomal polypeptide, wherein the at least one NA-binding domain is one or more of PUF, Cas, and/or an NA aptamer-binding domain. In advantageous embodiments, the fusion polypeptides may optionally further comprise additional regions, domains, sequences, and/or moieties endowing the polypeptide with particular functions. Non-limiting examples of additional domains comprised in the fusion polypeptide include (i) multimerization domains, (ii) linkers, (iii) release domains, (iv) RNA cleaving domains, (v) endosomal escape moieties, (vi) protease specific cleavage sites, (vii) inteins and/or (viii) targeting moieties.

Multimerization domains enable dimerization, trimerization, or any higher order of multimerization of the fusion polypeptides, which increases the sorting and trafficking of the fusion polypeptides into EVs and may also contribute to increase the yield of vesicles produced by EV-producing cells. Linkers are useful in providing increased flexibility to the fusion polypeptide constructs, and also to the corresponding polynucleotide constructs, and may also be used to ensure avoidance of steric hindrance and maintained functionality of the fusion polypeptides. Release domains may be included in the fusion polypeptide constructs in order to enable release of particular parts or domains from the original fusion polypeptide. This is particularly advantageous when the release of parts of the fusion polypeptide would increase bioactive delivery of the NA cargo and/or when a particular function of the fusion polypeptide works better when part of a smaller construct. Suitable release domains may be cis-cleaving sequences such as inteins, light induced monomeric or dimeric release domains such as Kaede, KikGR, EosFP, tdEosFP, mEos2, PSmOrange, the GFP-like Dendra proteins Dendra and Dendra2, CRY2-CIBN, etc. NA-cleaving domains may advantageously also be included in the fusion polypeptides, to trigger cleave of the NA cargo. Non-limiting examples of NA cleaving domains include endonucleases such as Cas6, Cas13, engineered PUF nucleases, site specific RNA nucleases etc. Furthermore, the fusion polypeptides of the present invention may also include endosomal escape domains to drive endosomal escape and thereby enhance the bioactive delivery of the EV *per se* and the EV NA cargo molecule. Another strategy for enhancing delivery is to target the EVs to cells, tissues, and/or organs or other bodily compartments. Targeting can be achieved by a variety of means, for instance the use of targeting peptides. Such targeting peptides may be anywhere from a few amino acids in length to 100s of amino acids in length, e.g. anywhere in the interval of 3-100 amino acids, 3-30 amino acids, 5-25 amino acids, e.g. 7 amino acids, 12 amino acids, 20 amino acids, etc. Targeting peptides of the present invention may also include full length proteins such as receptors, receptor ligands, etc. Furthermore, the targeting peptides as per the present invention may also include antibodies and antibody derivatives, e.g. monoclonal antibodies, single chain variable fragments (scFvs), other antibody domains, etc.

In a further aspect, the present invention relates to polynucleotide constructs encoding for the fusion polypeptides as per the present invention. The polynucleotide constructs may be present in various different forms and/or in different vectors. For instance, the polynucleotides may be essentially linear, circular, and/or has any secondary and/or tertiary and/or higher order structure. Furthermore, the present invention also relates to vectors comprising the polynucleotides, e.g. vectors such as plasmids, any circular DNA polynucleotide, mini-circles, viruses such as adenoviruses, adeno-associated viruses, lentivirus, mRNAs, and/or modified mRNAs.

The polynucleotide constructs as per the present invention may further comprise one or more sites or domains for imparting particular functionality into the polynucleotide. For example, the stability of the polynucleotide constructs can be enhanced through the use of stabilizing domains, such as polyA tails or stem loops, and the polynucleotide construct may also be controlled by particular promotors which may optionally be cell-type specific, inducible promotors, linkers, etc. The PolyA tail may optionally be inserted upstream of the Cas6 or Cas13 cut site so as to result in cleavage of mRNAs which retain the stabilizing PolyA tail.

The present invention further relates to various methods for producing EVs. Such methods may comprise the steps of (i) introducing into an EV-producing cell at least one polynucleotide construct, (ii) expressing in the EV-producing cell at least one polypeptide construct encoded for by the at least one polynucleotide construct, and optionally (iii) collecting from the EV-producing cell the EVs that are being produced, which comprises the polypeptide of interest from step (ii). In certain embodiments, a single polynucleotide construct is used whereas in other embodiments more than one polynucleotide construct is employed. Without wishing to be bound by any theory, it is surmised that the EV-producing cell into which a polynucleotide construct has been introduced (either transiently or stably, depending on the purpose and use of the EVs) produces EVs (such as exosomes) that comprise the polypeptide construct encoded for by the polynucleotide. The EVs may then optionally be collected, typically from the cell culture media, and optionally further purified before being put to a particular use. In advantageous embodiments, the EVs produced by said methods further comprise an NA cargo molecule, which is loaded into the EVs with the aid of the fusion polypeptide construct. Typically, a single EV comprises several copies of the NA cargo molecule but a single EV may also comprise more than one type of NA drug cargo molecule. As a non-limiting example of EVs comprising more than one type of NA drug cargo, a single EV (i.e. a population of a single type of EVs) may comprise e.g. an gRNA NA drug cargo molecule and an mRNA drug cargo molecule.

In further aspects, the present invention relates to cells comprising (i) at least one polynucleotide construct according and/or (ii) at least one polypeptide construct. Furthermore, the present invention also relates to cells comprising the EVs as per the present invention. The EV-producing cells may be present in the form of e.g. primary cells, cell lines, cells present in a multicellular organism, or essentially any other type of cell source and EV-producing cell material. The terms "source cell" or "EV source cell" or "parental cell" or "cell source" or "EV-producing cell" or any other similar terminology shall be understood to relate to any type of cell that is capable of producing EVs under suitable conditions, for instance in suspension culture or in adherent culture or any in other type of culturing system. Source cells as per the present invention may also include cells producing exosomes in vivo. The source cells per the present invention may be select from a wide range of cells and cell lines, for instance mesenchymal stem or stromal cells (obtainable from e.g. bone marrow, adipose tissue, Wharton's jelly, perinatal tissue, placenta, tooth buds, umbilical cord blood, skin tissue, etc.), fibroblasts, amnion cells and more specifically amnion epithelial cells optionally expressing various early markers, myeloid suppressor cells, M2 polarized macrophages, adipocytes, endothelial cells, fibroblasts, etc. Cell lines of particular interest include human umbilical cord endothelial cells (HUVECs), human embryonic kidney (HEK) cells, endothelial cell lines such as microvascular or lymphatic endothelial cells, erythrocytes, erythroid progenitors, chondrocytes, MSCs of different origin, amnion cells, amnion epithelial (AE) cells, any cells obtained through amniocentesis or from the placenta, airway or alveolar epithelial cells, fibroblasts, endothelial cells, etc. Also, immune cells such as B cells, T cells, NK cells, macrophages, monocytes, dendritic cells (DCs) are also within the scope of the present invention, and essentially any type of cell which is capable of producing EVs is also encompassed herein. Generally, EVs may be derived from essentially any cell source, be it a primary cell source or an immortalized cell line. The EV source cells may be any embryonic, fetal, and adult somatic stem cell types, including induced pluripotent stem cells (iPSCs) and other stem cells derived by any method. When treating neurological diseases, one may contemplate to utilize as source cells e.g. primary neurons, astrocytes, oligodendrocytes, microglia, and neural progenitor cells. The source cell may be either allogeneic, autologous, or even xenogeneic in nature to the patient to be treated, i.e. the cells may be from the patient himself or from an unrelated, matched or unmatched donor. In certain contexts, allogeneic cells may be preferable from a medical standpoint, as they could provide immuno-modulatory effects that may not be obtainable from autologous cells of a patient suffering from a certain indication. For instance, in the context of treating systemic, peripheral and/or neurological inflammation, allogeneic MSCs or AEs may be preferable as EVs obtainable from such cells may enable immuno-modulation via e.g. macrophage and/or neutrophil phenotypic switching (from pro-inflammatory M1 or N1 phenotypes to antiinflammatory M2 or N2 phenotypes, respectively).

As abovementioned, in preferred embodiments the EV-producing cells of the present invention are stably transfected and/or transduced with at least one polynucleotide construct(s) which encode(s) for (i) the fusion polypeptide comprising the NA-binding domain and (ii) the NA cargo molecule. In a highly preferred embodiment, the EV-producing cells are exposed to a clonal selection protocol allowing for clonal selection of a single cell clone. Thus, in highly preferred embodiments, the present invention relates to single cell clonal populations of EV-producing cells which are transfected and/or transduced to produce EVs comprising both the fusion polypeptide and the NA cargo molecule. The single clones may be obtained using limiting dilution methods, single-cell sorting, and/or isolation of individual cells using cloning cylinders.

In a further aspect, the present invention relates to *in vitro* methods for intracellular delivery of at least one RNA cargo molecule. Such methods may advantageously be carried out *in vitro* and/or *ex vivo.* The methods may comprise the steps of contacting a target cell with at least one EV as per the present invention, or more commonly a population of EVs as per the present invention. Furthermore, the methods for delivery of RNA cargo molecules as per the present invention may also comprise introducing into a cell present in any biological system (such as a human being) a polynucleotide encoding for the fusion polypeptides herein.

In additional aspects, the present invention relates to pharmaceutical compositions comprising either one of more of the following components: (i) at least one polynucleotide construct as described herein, (ii) at least one polypeptide construct as described herein, (iii) at least one EV as described herein, (iv) at least one cell as described herein, and/or (v) at least one population of EVs as described herein, typically formulated with a pharmaceutically acceptable excipient, carrier and/or diluent or similar. Furthermore, the present invention also pertains to the (i) at least one polynucleotide construct as described herein, (ii) at least one polypeptide construct as described herein, (iii) at least one EV as described herein, (iv) at least one cell as described herein, and/or (v) at least one population of EVs as described herein, and the (vi) above-mentioned pharmaceutical composition, for use in medicine. More specifically, the present invention relates to use in the prophylaxis and/or treatment and/or alleviation of a variety of diseases. Non-limiting examples of diseases and conditions include the following non-limiting examples: Crohn's disease, ulcerative colitis, ankylosing spondylitis, rheumatoid arthritis, multiple sclerosis, systemic lupus erythematosus, sarcoidosis, idiopathic pulmonary fibrosis, psoriasis, tumor necrosis factor (TNF) receptor-associated periodic syndrome (TRAPS), deficiency of the interleukin-1 receptor antagonist (DIRA), endometriosis, autoimmune hepatitis, scleroderma, myositis, stroke, acute spinal cord injury, vasculitis, Guillain-Barré syndrome, acute myocardial infarction, ARDS, sepsis, meningitis, encephalitis, liver failure, non-alcoholic steatohepatitis (NASH), non-alcoholic fatty liver disease (NAFLD), kidney failure, heart failure or any acute or chronic organ failure and the associated underlying etiology, graft-vs-host disease, Duchenne muscular dystrophy and other muscular dystrophies, lysosomal storage diseases such as Gaucher disease, Fabry's disease, MPS I, II (Hunter syndrome), and III, Niemann-Pick disease type A, B, and C, Pompe disease, etc., neurodegenerative diseases including Alzheimer's disease, Parkinson's disease, Huntington's disease and other trinucleotide repeat-related diseases, dementia, ALS, cancer-induced cachexia, anorexia, diabetes mellitus type 2, and various cancers. Virtually all types of cancer are relevant disease targets for the present invention, for instance, Acute lymphoblastic leukemia (ALL), Acute myeloid leukemia, Adrenocortical carcinoma, AIDS-related cancers, AIDS-related lymphoma, Anal cancer, Appendix cancer, Astrocytoma, cerebellar or cerebral, Basal-cell carcinoma, Bile duct cancer, Bladder cancer, Bone tumor, Brainstem glioma, Brain cancer, Brain tumor (cerebellar astrocytoma, cerebral astrocytoma/malignant glioma, ependymoma, medulloblastoma, supratentorial primitive neuroectodermal tumors, visual pathway and hypothalamic glioma), Breast cancer, Bronchial adenomas/carcinoids, Burkitt's lymphoma, Carcinoid tumor (childhood, gastrointestinal), Carcinoma of unknown primary, Central nervous system lymphoma, Cerebellar astrocytoma/Malignant glioma, Cervical cancer, Chronic lymphocytic leukemia, Chronic myelogenous leukemia, Chronic myeloproliferative disorders, Colon Cancer, Cutaneous T-cell lymphoma, Desmoplastic small round cell tumor, Endometrial cancer, Ependymoma, Esophageal cancer, Extracranial germ cell tumor, Extragonadal Germ cell tumor, Extrahepatic bile duct cancer, Eye Cancer (Intraocular melanoma, Retinoblastoma), Gallbladder cancer, Gastric (Stomach) cancer, Gastrointestinal Carcinoid Tumor, Gastrointestinal stromal tumor (GIST), Germ cell tumor (extracranial, extragonadal, or ovarian), Gestational trophoblastic tumor, Glioma (glioma of the brain stem, Cerebral Astrocytoma, Visual Pathway and Hypothalamic glioma), Gastric carcinoid, Hairy cell leukemia, Head and neck cancer, Heart cancer, Hepatocellular (liver) cancer, Hodgkin lymphoma, Hypopharyngeal cancer, Intraocular Melanoma, Islet Cell Carcinoma (Endocrine Pancreas), Kaposi sarcoma, Kidney cancer (renal cell cancer), Laryngeal Cancer, Leukemias ((acute lymphoblastic (also called acute lymphocytic leukemia), acute myeloid (also called acute myelogenous leukemia), chronic lymphocytic (also called chronic lymphocytic leukemia), chronic myelogenous (also called chronic myeloid leukemia), hairy cell leukemia)), Lip and Oral, Cavity Cancer, Liposarcoma, Liver Cancer (Primary), Lung Cancer (Non-Small Cell, Small Cell), Lymphomas, AIDS-related lymphoma, Burkitt lymphoma, cutaneous T-Cell lymphoma, Hodgkin lymphoma, Non-Hodgkin, Medulloblastoma, Merkel Cell Carcinoma, Mesothelioma, Metastatic Squamous Neck Cancer with Occult Primary, Mouth Cancer, Multiple Endocrine Neoplasia Syndrome, Multiple Myeloma/Plasma Cell Neoplasm, Mycosis Fungoides, Myelodysplastic/Myeloproliferative Diseases, Myelogenous Leukemia, Chronic Myeloid Leukemia (Acute, Chronic), Myeloma, Nasal cavity and paranasal sinus cancer, Nasopharyngeal carcinoma, Neuroblastoma, Oral Cancer, Oropharyngeal cancer, Osteosarcoma/malignant fibrous histiocytoma of bone, Ovarian cancer, Ovarian epithelial cancer (Surface epithelial-stromal tumor), Ovarian germ cell tumor, Ovarian low malignant potential tumor, Pancreatic cancer, Pancreatic islet cell cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Pleuropulmonary blastoma, Prostate cancer, Rectal cancer, Renal cell carcinoma (kidney cancer), Retinoblastoma, Rhabdomyosarcoma, Salivary gland cancer, Sarcoma (Ewing family of tumors sarcoma, Kaposi sarcoma, soft tissue sarcoma, uterine sarcoma), Sezary syndrome, Skin cancer (nonmelanoma, melanoma), Small intestine cancer, Squamous cell, Squamous neck cancer, Stomach cancer, Supratentorial primitive neuroectodermal tumor, Testicular cancer, Throat cancer, Thymoma and Thymic carcinoma, Thyroid cancer, Transitional cell cancer of the renal pelvis and ureter, Urethral cancer, Uterine cancer, Uterine sarcoma, Vaginal cancer, Vulvar cancer, Waldenström macroglobulinemia, and/or Wilm's tumor.

The EVs as per the present invention may be administered to a human or animal subject via various different administration routes, for instance auricular (otic), buccal, conjunctival, cutaneous, dental, electro-osmosis, endocervical, endosinusial, endotracheal, enteral, epidural, extra-amniotic, extracorporeal, hemodialysis, infiltration, interstitial, intra-abdominal, intra-amniotic, intra-arterial, intra-articular, intrabiliary, intrabronchial, intrabursal, intracardiac, intracartilaginous, intracaudal, intracavernous, intracavitary, intracerebral, intracisternal, intracorneal, intracoronal (dental), intracoronary, intracorporus cavernosum, intradermal, intradiscal, intraductal, intraduodenal, intradural, intraepidermal, intraesophageal, intragastric, intragingival, intraileal, intralesional, intraluminal, intralymphatic, intramedullary, intrameningeal, intramuscular, intraocular, intraovarian, intrapericardial, intraperitoneal, intrapleural, intraprostatic, intrapulmonary, intrasinal, intraspinal, intrasynovial, intratendinous, intratesticular, intrathecal, intrathoracic, intratubular, intratumor, intratym panic, intrauterine, intravascular, intravenous, intravenous bolus, intravenous drip, intraventricular, intravesical, intravitreal, iontophoresis, irrigation, laryngeal, nasal, nasogastric, occlusive dressing technique, ophthalmic, oral, oropharyngeal, other, parenteral, percutaneous, periarticular, peridural, perineural, periodontal, rectal, respiratory (inhalation), retrobulbar, soft tissue, subarachnoid, subconjunctival, subcutaneous, sublingual, submucosal, topical, transdermal, transmucosal, transplacental, transtracheal, transtympanic, ureteral, urethral, and/or vaginal administration, and/or any combination of the above administration routes, which typically depends on the disease to be treated and/or the characteristics of the EVs, the NA cargo molecule in question, or the EV population as such.

### Examples & experimental section

### Materials and methods

### • Construct design and cloning

Various NA-binding domains and variants thereof (e.g. PUF, mutated PUF, PUFx2, Cas6, mutated Cas6, Cas13, mutated Cas13, etc.) have been assessed, in combination with several exosomal polypeptides (such as CD81, CD63, CD9, syntenin, syndecan, Alix, CD133, etc.). ORFs were typically generated by synthesis and cloned into the mammalian expression vector pSF-CAG-Amp. Briefly, synthesized DNA and vector plasmid were digested with enzymes Notl and Sall as per manufacturers instruction (NEB). Restricted, purified DNA fragments were ligated together using T4 ligase as per manufacturers instruction (NEB). Successful ligation events were selected for by bacterial transformation on ampicillin-supplemented plates. Plasmid for transfection was generated by 'maxi-prep', as per manufacturers instruction.

### • Cell culture and transfection

Depending on the experimental design and assays, in certain cases, non-viral transient transfection and EV production was carried out in conventional 2D cell culture, whereas in other cases virus-mediated transduction was employed to create stable cell lines, which were typically cultured in bioreactors and/or shaking incubators of different types. For conciseness, only a few examples are mentioned herein.

HEK293T cells were typically seeded into 15 cm dishes (9×10⁶ cells per dish) and left overnight in serum-containing DMEM as recommended by ATCC. The following day the cells were transiently transfected with lipoplexed DNA added directly onto cells. Briefly, DNA and polyethyleneimine (PEI) were separately incubated in OptiMEM for 5 minutes before combining together for 20 minutes at room temperature. Lipoplexed DNA and cells were co-incubated for 6 hours following which conditioned culture media was changed to OptiMEM for 48 hours. Other cells and cell lines that were evaluated in dishes, flasks and other cell culture vessels included bone marrow-derived mesenchymal stromal cells (BM-MSCs) and Wharton's jelly-derived MSCs (WJ-MSCs), amnion cells, amnion epithelial cells, fibroblasts, various endothelial and epithelial cells, as well as various immune cells and cell lines.

In the case of viral transduction and creation of stable cell lines for various combinations fusion polypeptide constructs and NA cargo molecules, cell sources such as BM-MSCs, WJ-MSC, fibroblasts, amnion epithelial cells, fibroblasts, various endothelial and epithelial cells, were virus-transduced, typically using lentivirus (LV) transduction. Typically, 24 hours before infection, 100.000 cells (e.g. fibroblasts, MSCs, etc.) or 200.000 cells (e.g. HEK293T) are plated in a 6-well plate. 2 uL of LV and optionally Polybrene (or hexadimethrine bromide, final concentration on the well of 8 ug/mL) are added, and 24 hours post transduction the cell medium of transduced cells is changed to fresh complete media. At 72 hours post transduction, puromycin selection (4-6µg/ml) is performed, normally for 7 days followed by analysis of stable expression of the fusion polypeptides.

Stable cells were cultured in either 2D culture or in bioreactors and conditioned media was subsequently harvested for exosome preparation. Various preparation and purification steps were carried out. The standard workflow comprises the steps of pre-clearing of the supernatant, filtration-based concentration, chromatography-based removal of protein contaminants, and optional formulation of the resultant exosome composition in a suitable buffer for *in vitro* and/or *in vivo* assays.

### • Assays and analytics

Western blot is a highly convenient analytical method to evaluate the enrichment of Pols in EVs. Briefly, SDS-PAGE was performed according to manufacturer's instruction (Invitrogen, Novex PAGE 4-12% gels), whereby 1 × 10¹⁰ exosomes and 20 ug cell lysate were loaded per well. Proteins from the SDS-PAGE gel were transferred to PVDF membrane according to manufacturer's instruction (immobilon (RTM), Invitrogen). Membranes were blocked in Odyssey blocking buffer (Licor) and probed with antibodies against NA-binding domain polypeptide and/or the exosomal protein according to supplier's instruction (Primary antibodies ― Abcam, Secondary antibodies - Licor). Molecular probes visualized at 680 and 800 nm wavelengths.

For EV size determination, nanoparticle tracking analysis (NTA) was performed with a NanoSight (RTM) instrument equipped with analytical software, or occasionally with a Particle Matrics machine. For recordings on the NanoSight (RTM), a camera level of 13 or 15 and automatic function for all post-acquisition settings were used. Electron microscopy and fluorescence microscopy were frequently used to validate and assess EV morphology and size.

EVs were isolated and purified using a variety of methods, typically a combination of filtration such as TFF and size exclusion liquid chromatography and/or bead-elute liquid chromatography. Typically, EV-containing media was collected and subjected to a low speed spin at 300g for 5 minutes, followed by 2000*g* spin for 10 minutes to remove larger particles and cell debris. The supernatant was then filtered with a 0.22 µm syringe filter and subjected to different purification steps. Large volumes were diafiltrated and concentrated to roughly 20 ml using the Vivaflow 50R tangential flow (TFF) device (Sartorius) with 100 kDa cutoff filters or the KR2i TFF system (SpectrumLabs) with 100 or 300 kDa cutoff hollow fibre filters. The preconcentrated medium was subsequently loaded onto the bead-eluate columns (HiScreen (RTM) or HiTrap (RTM) Capto Core 700 column, GE Healthcare Life Sciences), connected to an ÄKTAprime (RTM) plus or ÄKTA Pure 25 (RTM) chromatography system (GE Healthcare Life Sciences). Flow rate settings for column equilibration, sample loading and column cleaning in place procedure were chosen according to the manufacturer's instructions. The sample was collected according to the UV absorbance chromatogram and concentrated using an Amicon Ultra-15 10 kDa molecular weight cut-off spin-filter (Millipore) to a final volume of 100 µl and stored at -80°C for further downstream analysis. To assess the protein and RNA elution profiles, media was concentrated and diafiltrated with KR2i TFF system using 100 kDa and 300 kDa hollow fibre filters and a sample analysed on a Tricorn 10/300 Sepharose 4 Fast Flow (S4FF) column (GE Healthcare Life Sciences).

### • Examples

*Example 1*. Bone marrow-derived MSCs were cultured in conventional tissue culture flasks and transiently transfected using PEI transfection to enable loading and expression of mRNA cargo molecules and fusion polypeptide constructs. Figure 2 shows loading in EVs obtained from the BM-MSCs of mRNA cargo molecules encoding NanoLuc (RTM) and p21. The loading was achieved using fusion polypeptide constructs comprising CD63 as the exosomal polypeptide and PUF or Cas6 as the NA-binding domains. The experiment also included varying numbers of binding sites for the NA-binding domains, namely 0, 3 and 6 binding sites, which were inserted in different places to the 3' and/or 5' flanks of the coding region.

The MSC-EVs were purified using a sequential combination of TFF and SEC. Expression of only the exosomal polypeptide CD63 did not result in loading of any mRNA into the EVs (right set of columns in the graph in Figure 2). Expression of fusion polypeptides comprising PUF (left set of columns: two PUFs domain flanking CD63 both N terminally and C terminally, i.e. 4 PUF constructs in total) (second set of columns from left: one PUF domain flanking CD63 both N terminally and C terminally) and mutated Cas6 (second from right) did result in significant mRNA loading of both NanoLuc (RTM) and p21 mRNAs upon expression in the EV source cells. The loading of NanoLuc (RTM) was overall more efficient than the loading of p21, with up to around 45 copies of mRNA per EV.

*Example 2.* Silencing of gene expression *in vitro* in target cells (HEK293 cells) upon EV-mediated delivery of an shRNA targeting huntingtin. Amnion cells cultured in a shaking incubator were transduced using lentiviral transduction to secrete EVs comprising the shRNA and fusion polypeptide constructs comprising as NA-binding domain either PUF or Cas6, combined with CD63 or CD81 as the EV polypeptides, respectively. EVs were purified from the supernatant using ultracentrifugation. The Y axis of Figure 3 shows how the huntingtin expression level is decreased with increasing numbers of binding sites in the NA cargo molecule, resulting in approximately 80% knockdown using a fusion polypeptide comprising PUF-CD63-PUF to load the anti-huntingtin shRNA.

*Example 3.* Expression of NanoLuc (RTM) as a reporter system in target Huh7 cells after HEK EV-mediated delivery of a NanoLuc (RTM) mRNA. HEK293T cells were stably transduced to express various fusion polypeptide constructs for loading of reporter NanoLuc (RTM) mRNA cargo into EVs. The NanoLuc (RTM) mRNA cargo molecule was engineered to comprise 0, 3, or 6 binding sites for the NA-binding domains comprising the fusion polypeptide constructs, in this case PUFx2-CD63-PUFx2 (two PUF NA-binding polypeptides inserted both N terminally and C terminally of the exosomal polypeptide CD63), PUF-CD63-PUF, and Cas6-CD9-Cas6 (Figure 4).

Post purification of the HEK-derived EVs based on TFF combined with bead-elute LC the EVs were added to HEK cells in at optimal concentration, which for this assay was 10^7 EVs per well in 6-well plates of Huh7 target cells. The Y axis of Figure 4 shows relative light (luminescence) units (RLU) normalized over µg of protein, indicating enhanced delivery and/or translation with increasing numbers of binding sites using the different fusion polypeptide constructs. Figure 4, therefore, demonstrates that the present invention provides EVs which are capable of delivering bioactive mRNA to cells which is then successfully translated by these cells. This is a significant advantage of the present invention over the prior art which is only able to load EVs with RNA but is not able to deliver those RNAs to the cytosol of target cells to be actively translated.

### Embodiments of the invention are set out in the following clauses:

1. An extracellular vesicle (EV) comprising at least one fusion polypeptide comprising at least one nucleic acid (NA)-binding domain and at least one exosomal polypeptide, wherein the at least one NA-binding domain is one or more of PUF, Cas6, Cas13, and/or an NA aptamer-binding domain.
2. The EV according to clause 1, further comprising at least one NA cargo molecule.
3. The EV according to clause 2, wherein the at least one NA cargo molecule is transported into the EV with the help of the fusion polypeptide.
4. The EV according to any one of clauses 2-3, wherein the NA cargo molecule is selected from the group comprising shRNA, miRNA, mRNA, gRNA, pri-miRNA, pre-miRNA, circular RNA, piRNA, tRNA, rRNA, snRNA, IncRNA, ribozymes, mini-circle DNA, and/or plasmid DNA.
5. The EV according to any one of clauses 2-4, wherein each NA cargo molecule comprises (i) at least one binding site for the NA-binding domain and (ii) a therapeutic polynucleotide domain.
6. The EV according to clause 5, wherein the NA cargo molecule comprises a cleavage site between the at least one binding site and the therapeutic polynucleotide domain.
7. The EV according to any one of clauses 2-6, wherein the NA cargo molecule encodes for a therapeutic polypeptide.
8. The EV according to clause 7, wherein the therapeutic polypeptide is selected from the group comprising antibodies, intrabodies, single chain variable fragments, affibodies, enzymes, transporters, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, nucleotide binding proteins, heat shock proteins, CRISPR-associated proteins, and any combination thereof.
9. The EV according to any one of the preceding clauses, wherein the fusion polypeptide comprises at least one exosomal polypeptide flanked N- and/or C-terminally by NA-binding domains and/or wherein the at least one NA-binding domain is inserted into the exosomal polypeptide sequence.
10. The EV according to any one of the preceding clauses, wherein the exosomal polypeptide is selected from the group comprising CD9, CD53, CD63, CD81, CD54, CD50, FLOT1, FLOT2, CD49d, CD71, CD133, CD138, CD235a, ALIX, AARDC1, Syntenin-1, Syntenin-2, Lamp2b, TSPAN8, syndecan-1, syndecan-2, syndecan-3, syndecan-4, TSPAN14, CD37, CD82, CD151, CD231, CD102, NOTCH1, NOTCH2, NOTCH3, NOTCH4, DLL 1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, Fc receptors, interleukin receptors, immunoglobulins, MHC-I or MHC-II components, CD2, CD3 epsilon, CD3 zeta, CD13, CD18, CD19, CD30, CD34, CD36, CD40, CD40L, CD44, CD45, CD45RA, CD47, CD86, CD110, CD111, CD115, CD117, CD125, CD135, CD184, CD200, CD279, CD273, CD274, CD362, COL6A1, AGRN, EGFR, GAPDH, GLUR2, GLUR3, HLA-DM, HSPG2, L1CAM, LAMB1, LAMC1, LFA-1, LGALS3BP, Mac-1 alpha, Mac-1 beta, MFGE8, SLIT2, STX3, TCRA, TCRB, TCRD, TCRG, VTI1A, VTI1B, other exosomal polypeptides, and any combinations thereof.
11. The EV according to any one of the preceding clauses, wherein the EV further comprises at least one targeting moiety.
12. The EV according to any one of clauses 2-11, wherein the NA cargo molecule is linear, circularized, and/or has any secondary and/or tertiary and/or other structure.
13. The EV according to any one of clauses 2-12, wherein the NA cargo molecule comprises one or more of the following:
   (i) a site for miRNA binding, wherein such site optionally is tissue and/or cell type specific;
   (ii) at least one stabilizing domain, such as a polyA tail or a stem loop; or,
   (iii) at least one hybrid UTR in the 5' and/or 3' end.
14. A population of EVs according to any one of the preceding clauses.
15. The population according to clause 14, wherein the average number of NA cargo molecules per EV throughout the population of EVs is above one per EV.
16. The population according to any one of clauses 14-15, wherein the population comprises at least two subpopulations,
   wherein the first EV subpopulation comprises on average more than one fusion polypeptide per EV, and
   wherein the second EV subpopulation comprises the NA cargo molecule in question combined with on average more than one fusion polypeptide per EV.
17. The population according to any one of clauses 14-16, wherein at least 5%, at least 10%, at least 20%, at least 50%, at least 70%, at least 75%, at least 80%, at least 85%, at least 90%, and/or at least 95% of all EVs comprise at least one NA cargo molecule.
18. A polypeptide construct comprising a fusion polypeptide comprising at least one NA-binding domain and at least one exosomal polypeptide, wherein the at least one NA-binding domain is one or more of PUF, Cas6, Cas13, and/or an NA aptamer-binding domain.
19. The polypeptide construct according to clause 18, optionally further comprising one or more of:
   (i) at least one multimerization domain;
   (ii) at least one linker;
   (iii) at least one release domain such as an intein;
   (iv) at least one RNA cleaving domain;
   (v) at least one endosomal escape moiety, and/or,
   (vi) at least one targeting moiety.
20. A polynucleotide construct encoding for the polypeptide construct according to any one of clauses 18-19.
21. The polynucleotide construct according to clause 20, wherein the polynucleotide is essentially linear, circularized, and/or has any secondary and/or tertiary and/or higher order structure and/or is comprised in a vector such as a plasmid, a circular DNA polynucleotide, a virus, an adeno-associated virus, an mRNA, a modified mRNA, and/or a mini-circle.
22. A method for producing EVs according to any one of the preceding clauses, comprising:
   (i) introducing into an EV-producing cell at least one polynucleotide construct according to any one of clauses 20-21;
   (ii) expressing in the EV-producing cell at least one polypeptide construct encoded for by the at least one polynucleotide construct, thereby generating said EVs.
23. A cell comprising (i) at least one polynucleotide construct according to any one of clauses 20-21 and/or (ii) at least one polypeptide construct according to any one of clauses 18-19 and/or (iii) at least one EV according to any one of clauses 1-13.
24.The cell according to clause 23, wherein the cell is a monoclonal cell and/or a monoclonal cell line.
25. The cell according to any one of clauses 23-24, wherein the cell is stably modified to comprise at least one monocistronic, bicistronic or multicistronic polynucleotide construct encoding for the polypeptide construct according to any one of clauses 18-19 and at least one NA cargo molecule.
26. An *in vitro* method for intracellular delivery of at least one RNA cargo molecule, comprising contacting a target cell with at least one EV according to any one of clauses 1-13 and/or at least one population of EVs according to any one of clauses 14-17 and/or at least one polynucleotide construct according to any one of clauses 20-21 .
27. A pharmaceutical composition comprising (i) at least one polynucleotide construct according to any one of clauses 20-21, (ii) at least one polypeptide construct according to any one of clauses 18-19, (iii) at least one EV according to any one of clauses 1-13, (iv) at least one cell according to any one of clauses 23-25, and/or (v) at least one population of EVs according to any one of clauses 14-17, and a pharmaceutically acceptable excipient or carrier.
28. The (i) at least one polynucleotide construct according to any one of clauses 20-21, (ii) at least one polypeptide construct according to any one of clauses 18-19, (iii) at least one EV according to any one of clauses 1-13, (iv) at least one cell according to any one of clauses 23-25, (v) at least one population of EVs according to any one of clauses 14-17, and/or (vi) the pharmaceutical composition according to clause 27, for use in medicine.

## Claims

1. A cell comprising at least one polynucleotide construct encoding a polypeptide construct comprising a fusion polypeptide, wherein the fusion polypeptide comprises at least one nucleic acid (NA)-binding domain and at least one exosomal polypeptide, wherein the at least one NA-binding domain is one or more of Pumilio and FBF homology protein (PUF), a CRISPR-associated polypeptide (Cas) and/or an NA aptamer-binding domain.

2. A cell according to claim 1, wherein the cell is a mesenchymal stromal cell, mesenchymal stem cell, Wharton's jelly-derived mesenchymal stem cell, amnion cell, amnion epithelial cell, fibroblast, human umbilical cord endothelial cell (HUVEC), human embryonic kidney (HEK) cell or endothelial cell, optionally wherein the cell is a monoclonal cell and/or a monoclonal cell line.

3. A cell according to claim 1 or claim 2, wherein the cell is stably modified to comprise the at least one polynucleotide construct, and the at least one polynucleotide construct is a monocistronic, bicistronic or multicistronic polynucleotide construct encoding the polypeptide construct and at least one NA cargo molecule.

4. A cell according to any one of claims 1-3, wherein the at least one polynucleotide construct is essentially linear, circularized and/or has any secondary and/or tertiary and/or higher order structure, and/or is comprised in a vector, optionally a plasmid, a circular DNA polynucleotide, a virus, an adeno-associated virus, an mRNA, a modified mRNA and/or a mini-circle.

5. A cell according to any one of claims 1-4, wherein the at least one NA-binding domain includes a Cas selected from Cas6, Cas9 and Cas13, and/or a PUF protein having the sequence set forth in SEQ ID NO:1, SEQ ID NO:2 or SEQ ID NO:3.

6. A cell according to any one of claims 1-5, wherein the fusion polypeptide comprises more than one NA-binding domain.

7. A cell according to any one of claims 1-6, wherein the at least one exosomal polypeptide is selected from the group consisting of CD9, CD53, CD63, CD81, CD54, CD50, FLOT1, FLOT2, CD49d, CD71, CD133, CD138 (syndecan-1), CD235a, ALIX, AARDC1, Syntenin-1, Syntenin-2, Lamp2b, TSPAN8, syndecan-2, syndecan-3, syndecan-4, TSPAN14, CD37, CD82, CD151, CD231, CD102, NOTCH1, NOTCH2, NOTCH3, NOTCH4, DLL1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, Fc receptors, interleukin receptors, immunoglobulins, MHC-I or MHC-II components, CD2, CD3 epsilon, CD3 zeta, CD13, CD18, CD19, CD30, TSG101, CD34, CD36, CD40, CD40L, CD44, CD45, CD45RA, CD47, CD86, CD110, CD111, CD115, CD117, CD125, CD135, CD184, CD200, CD279, CD273, CD274, CD362, COL6A1, AGRN, EGFR, GAPDH, GLUR2, GLUR3, HLA-DM, HSPG2, L1CAM, LAMB1, LAMC1, LFA-1, LGALS3BP, Mac-1 alpha, Mac-1 beta, MFGE8, SLIT2, STX3, TCRA, TCRB, TCRD, TCRG, VTI1A, VTI1B, and any combinations thereof.

8. A cell according to any one of claims 1-7, wherein the fusion polypeptide further comprises:
(i) at least one multimerization domain;
(ii) at least one linker;
(iii) at least one release domain;
(iv) at least one RNA cleaving domain;
(v) at least one endosomal escape moiety,
(vi) at least one protease specific cleavage site;
(vii) at least one intein; and/or,
(viii) at least one targeting moiety.

9. A cell according to any one of claims 1-8, wherein the at least one polynucleotide construct further encodes at least one NA cargo molecule, or the cell further comprises a polynucleotide construct encoding at least one NA cargo molecule.

10. A cell according to claim 9, wherein the at least one NA cargo molecule is selected from the group consisting of shRNA, miRNA, mRNA, gRNA, pri-miRNA, pre-miRNA, circular RNA, piRNA, tRNA, rRNA, snRNA, IncRNA, ribozymes, mini-circle DNA and/or plasmid DNA.

11. A cell according to claim 9 or claim 10, wherein the at least one NA cargo molecule comprises (i) at least one binding site for the NA-binding domain of the fusion polypeptide, and (ii) a therapeutic polynucleotide domain, and optionally further comprises a cleavage site between the at least one binding site and the therapeutic polynucleotide domain.

12. A cell according to any one of claims 9-11, wherein the at least one NA cargo molecule comprises a plurality of binding sites for the NA-binding domain.

13. A cell according to any one of claims 9-12, wherein the at least one NA cargo molecule encodes a therapeutic polypeptide, optionally wherein the therapeutic polypeptide is selected from the group consisting of antibodies, intrabodies, single chain variable fragments, affibodies, enzymes, transporters, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, nucleotide binding proteins, heat shock proteins, CRISPR-associated proteins, cytokines, cytokine receptors, caspases and any combination thereof.

14. A cell according to any one of claims 9-13, wherein the NA cargo molecule:
(a) is linear or circularized;
(b) has secondary and/or tertiary structure; and/or
(c) comprises one or more of the following:
(i) a site for miRNA binding, wherein such site optionally is tissue and/or cell type specific;
(ii) at least one stabilizing domain, optionally selected from a long polyA tail or more than one polyA tail;
(iii) at least one stem loop structure in the 5' and/or 3' UTR;
(iv) an RNA polymerase;
(v) codon-optimized sequences;
(vi) at least one hybrid UTR in the 5' and/or 3' end; and
(vii) at least one ribozyme.

15. An *in vitro* method for producing EVs, comprising:
i) introducing into an EV-producing cell at least one polynucleotide construct encoding a polypeptide construct comprising a fusion polypeptide, wherein the fusion polypeptide comprises at least one NA-binding domain and at least one exosomal polypeptide, wherein the at least one NA-binding domain is one or more of PUF, a Cas and/or an NA aptamer-binding domain;
ii) expressing in the EV-producing cell the polypeptide construct; and optionally
iii) collecting from the EV-producing cell the EVs so produced, said EVs comprising the at least one polypeptide construct.


SEQUENCE LISTING

<110>  Evox Therapeutics Ltd

<120>  Exosomes comprising RNA therapeutics

<130>  Evox12

<150>  GB1718471.4
<151>  2017-11-08

<160>  7

<170>  PatentIn version 3.5

<210>  1
<211>  349
<212>  PRT
<213>  Artificial Sequence

<220>
<223>  PUF 531

<400>  1

Gly Arg Ser Arg Leu Leu Glu Asp Phe Arg Asn Asn Arg Tyr Pro Asn
1               5                   10                  15

Leu Gln Leu Arg Glu Ile Ala Gly His Ile Met Glu Phe Ser Gln Asp
            20                  25                  30

Gln His Gly Ser Arg Phe Ile Glu Leu Lys Leu Glu Arg Ala Thr Pro
        35                  40                  45

Ala Glu Arg Gln Leu Val Phe Asn Glu Ile Leu Gln Ala Ala Tyr Gln
    50                  55                  60

Leu Met Val Asp Val Phe Gly Asn Tyr Val Ile Gln Lys Phe Phe Glu
65                  70                  75                  80

Phe Gly Ser Leu Glu Gln Lys Leu Ala Leu Ala Glu Arg Ile Arg Gly
                85                  90                  95

His Val Leu Ser Leu Ala Leu Gln Met Tyr Gly Ser Arg Val Ile Glu
            100                 105                 110

Lys Ala Leu Glu Phe Ile Pro Ser Asp Gln Gln Asn Glu Met Val Arg
        115                 120                 125

Glu Leu Asp Gly His Val Leu Lys Cys Val Lys Asp Gln Asn Gly Asn
    130                 135                 140

His Val Val Gln Lys Cys Ile Glu Cys Val Gln Pro Gln Ser Leu Gln
145                 150                 155                 160

Phe Ile Ile Asp Ala Phe Lys Gly Gln Val Phe Ala Leu Ser Thr His
                165                 170                 175

Pro Tyr Gly Ser Arg Val Ile Glu Arg Ile Leu Glu His Cys Leu Pro
            180                 185                 190

Asp Gln Thr Leu Pro Ile Leu Glu Glu Leu His Gln His Thr Glu Gln
        195                 200                 205

Leu Val Gln Asp Gln Tyr Gly Asn Tyr Val Ile Gln His Val Leu Glu
    210                 215                 220

His Gly Arg Pro Glu Asp Lys Ser Lys Ile Val Ala Glu Ile Arg Gly
225                 230                 235                 240

Asn Val Leu Val Leu Ser Gln His Lys Phe Ala Ser Asn Val Val Glu
                245                 250                 255

Lys Cys Val Thr His Ala Ser Arg Thr Glu Arg Ala Val Leu Ile Asp
            260                 265                 270

Glu Val Cys Thr Met Asn Asp Gly Pro His Ser Ala Leu Tyr Thr Met
        275                 280                 285

Met Lys Asp Gln Tyr Ala Asn Tyr Val Val Gln Lys Met Ile Asp Val
    290                 295                 300

Ala Glu Pro Gly Gln Arg Lys Ile Val Met His Lys Ile Arg Pro His
305                 310                 315                 320

Ile Ala Thr Leu Arg Lys Tyr Thr Tyr Gly Lys His Ile Leu Ala Lys
                325                 330                 335

Leu Glu Lys Tyr Tyr Met Lys Asn Gly Val Asp Leu Gly
            340                 345

<210> 2
<211> 349
<212> PRT
<213> Artificial Sequence

<220>
<223> PUF mRNA loc

<400> 2

Gly Arg Ser Arg Leu Leu Glu Asp Phe Arg Asn Asn Arg Tyr Pro Asn
1               5                   10                  15

Leu Gln Leu Arg Glu Ile Ala Gly His Ile Met Glu Phe Ser Gln Asp
            20                  25                  30

Gln His Gly Ser Arg Phe Ile Gln Leu Lys Leu Glu Arg Ala Thr Pro
        35                  40                  45

Ala Glu Arg Gln Leu Val Phe Asn Glu Ile Leu Gln Ala Ala Tyr Gln
    50                  55                  60

Leu Met Val Asp Val Phe Gly Asn Tyr Val Ile Gln Lys Phe Phe Glu
65                  70                  75                  80

Phe Gly Ser Leu Glu Gln Lys Leu Ala Leu Ala Glu Arg Ile Arg Gly
                85                  90                  95

His Val Leu Ser Leu Ala Leu Gln Met Tyr Gly Cys Arg Val Ile Gln
            100                 105                 110

Lys Ala Leu Glu Phe Ile Pro Ser Asp Gln Gln Asn Glu Met Val Arg
        115                 120                 125

Glu Leu Asp Gly His Val Leu Lys Cys Val Lys Asp Gln Asn Gly Asn
    130                 135                 140

His Val Val Gln Lys Cys Ile Glu Cys Val Gln Pro Gln Ser Leu Gln
145                 150                 155                 160

Phe Ile Ile Asp Ala Phe Lys Gly Gln Val Phe Ala Leu Ser Thr His
                165                 170                 175

Pro Tyr Gly Cys Arg Val Ile Gln Arg Ile Leu Glu His Cys Leu Pro
            180                 185                 190

Asp Gln Thr Leu Pro Ile Leu Glu Glu Leu His Gln His Thr Glu Gln
        195                 200                 205

Leu Val Gln Asp Gln Tyr Gly Ser Tyr Val Ile Glu His Val Leu Glu
    210                 215                 220

His Gly Arg Pro Glu Asp Lys Ser Lys Ile Val Ala Glu Ile Arg Gly
225                 230                 235                 240

Asn Val Leu Val Leu Ser Gln His Lys Phe Ala Asn Asn Val Val Gln
                245                 250                 255

Lys Cys Val Thr His Ala Ser Arg Thr Glu Arg Ala Val Leu Ile Asp
            260                 265                 270

Glu Val Cys Thr Met Asn Asp Gly Pro His Ser Ala Leu Tyr Thr Met
        275                 280                 285

Met Lys Asp Gln Tyr Ala Asn Tyr Val Val Gln Lys Met Ile Asp Val
    290                 295                 300

Ala Glu Pro Gly Gln Arg Lys Ile Val Met His Lys Ile Arg Pro His
305                 310                 315                 320

Ile Ala Thr Leu Arg Lys Tyr Thr Tyr Gly Lys His Ile Leu Ala Lys
                325                 330                 335

Leu Glu Lys Tyr Tyr Met Lys Asn Gly Val Asp Leu Gly
            340                 345

<210>  3
<211>  644
<212>  PRT
<213>  Artificial Sequence

<220>
<223>  PUFx2

<400>  3

Gly Arg Ser Arg Leu Leu Glu Asp Phe Arg Asn Asn Arg Tyr Pro Asn
1               5                   10                  15

Leu Gln Leu Arg Glu Ile Ala Gly His Ile Met Glu Phe Ser Gln Asp
            20                  25                  30

Gln His Gly Ser Arg Phe Ile Gln Leu Lys Leu Glu Arg Ala Thr Pro
        35                  40                  45

Ala Glu Arg Gln Leu Val Phe Asn Glu Ile Leu Gln Ala Ala Tyr Gln
    50                  55                  60

Leu Met Val Asp Val Phe Gly Asn Tyr Val Ile Gln Lys Phe Phe Glu
65                  70                  75                  80

Phe Gly Ser Leu Glu Gln Lys Leu Ala Leu Ala Glu Arg Ile Arg Gly
                85                  90                  95

His Val Leu Ser Leu Ala Leu Gln Met Tyr Gly Cys Arg Val Ile Gln
            100                 105                 110

Lys Ala Leu Glu Phe Ile Pro Ser Asp Gln Gln Asn Glu Met Val Arg
        115                 120                 125

Glu Leu Asp Gly His Val Leu Lys Cys Val Lys Asp Gln Asn Gly Asn
    130                 135                 140

His Val Val Gln Lys Cys Ile Glu Cys Val Gln Pro Gln Ser Leu Gln
145                 150                 155                 160

Phe Ile Ile Asp Ala Phe Lys Gly Gln Val Phe Ala Leu Ser Thr His
                165                 170                 175

Pro Tyr Gly Cys Arg Val Ile Gln Arg Ile Leu Glu His Cys Leu Pro
            180                 185                 190

Asp Gln Thr Leu Pro Ile Leu Glu Glu Leu His Gln His Ile Met Glu
        195                 200                 205

Phe Ser Gln Asp Gln His Gly Ser Arg Phe Ile Gln Leu Lys Leu Glu
    210                 215                 220

Arg Ala Thr Pro Ala Glu Arg Gln Leu Val Phe Asn Glu Ile Leu Gln
225                 230                 235                 240

Ala Ala Tyr Gln Leu Met Val Asp Val Phe Gly Asn Tyr Val Ile Gln
                245                 250                 255

Lys Phe Phe Glu Phe Gly Ser Leu Glu Gln Lys Leu Ala Leu Ala Glu
            260                 265                 270

Arg Ile Arg Gly His Val Leu Ser Leu Ala Leu Gln Met Tyr Gly Cys
        275                 280                 285

Arg Val Ile Gln Lys Ala Leu Glu Phe Ile Pro Ser Asp Gln Gln Asn
    290                 295                 300

Glu Met Val Arg Glu Leu Asp Ser His Val Leu Lys Cys Val Lys Asp
305                 310                 315                 320

Gln Asn Gly Asn His Val Val Gln Lys Cys Ile Glu Cys Val Gln Pro
                325                 330                 335

Gln Ser Leu Gln Phe Ile Ile Asp Ala Phe Lys Gly Gln Val Phe Ala
            340                 345                 350

Leu Ser Thr His Pro Tyr Gly Cys Arg Val Ile Gln Arg Ile Leu Glu
        355                 360                 365

His Cys Leu Pro Asp Gln Thr Leu Pro Ile Leu Glu Glu Leu His Gln
    370                 375                 380

His Thr Glu Gln Leu Val Gln Asp Gln Tyr Gly Asn Tyr Val Ile Gln
385                 390                 395                 400

His Val Leu Glu His Gly Arg Pro Glu Asp Lys Ser Lys Ile Val Ala
                405                 410                 415

Glu Ile Arg Gly Asn Val Leu Val Leu Ser Gln His Lys Phe Ala Ser
            420                 425                 430

Asn Val Val Glu Lys Cys Val Thr His Ala Ser Arg Thr Glu Arg Ala
        435                 440                 445

Val Leu Ile Asp Glu Val Cys Thr Met Asn Asp Gly Pro His Ser Ala
    450                 455                 460

Leu Tyr Thr Met Met Lys Asp Gln Tyr Ala Asn Tyr Val Val Gln Lys
465                 470                 475                 480

Met Ile Asp Val Ala Glu Pro Gly Gln Arg Lys Ile Val Met His Glu
                485                 490                 495

Leu His Gln His Thr Glu Gln Leu Val Gln Asp Gln Tyr Gly Asn Tyr
            500                 505                 510

Val Ile Gln His Val Leu Glu His Gly Arg Pro Glu Asp Lys Ser Lys
        515                 520                 525

Ile Val Ala Glu Ile Arg Gly Asn Val Leu Val Leu Ser Gln His Lys
    530                 535                 540

Phe Ala Ser Asn Val Val Glu Lys Cys Val Thr His Ala Ser Arg Thr
545                 550                 555                 560

Glu Arg Ala Val Leu Ile Asp Glu Val Cys Thr Met Asn Asp Gly Pro
                565                 570                 575

His Ser Ala Leu Tyr Thr Met Met Lys Asp Gln Tyr Ala Asn Tyr Val
            580                 585                 590

Val Gln Lys Met Ile Asp Val Ala Glu Pro Gly Gln Arg Lys Ile Val
        595                 600                 605

Met His Lys Ile Arg Pro His Ile Ala Thr Leu Arg Lys Tyr Thr Tyr
    610                 615                 620

Gly Lys His Ile Leu Ala Lys Leu Glu Lys Tyr Tyr Met Lys Asn Gly

625                 630                 635                 640

Val Asp Leu Gly

<210>  4
<211>  186
<212>  PRT
<213>  Artificial Sequence

<220>
<223>  Cas6

<400>  4

Asp His Tyr Leu Asp Ile Arg Leu Arg Pro Asp Pro Glu Phe Pro Pro
1               5                   10                  15

Ala Gln Leu Met Ser Val Leu Phe Gly Lys Leu His Gln Ala Leu Val
            20                  25                  30

Ala Gln Gly Gly Asp Arg Ile Gly Val Ser Phe Pro Asp Leu Asp Glu
        35                  40                  45

Ser Arg Ser Arg Leu Gly Glu Arg Leu Arg Ile His Ala Ser Ala Asp
    50                  55                  60

Asp Leu Arg Ala Leu Leu Ala Arg Pro Trp Leu Glu Gly Leu Arg Asp
65                  70                  75                  80

His Leu Gln Phe Gly Glu Pro Ala Val Val Pro His Pro Thr Pro Tyr
                85                  90                  95

Arg Gln Val Ser Arg Val Gln Ala Lys Ser Asn Pro Glu Arg Leu Arg
            100                 105                 110

Arg Arg Leu Met Arg Arg His Asp Leu Ser Glu Glu Glu Ala Arg Lys
        115                 120                 125

Arg Ile Pro Asp Thr Val Ala Arg Ala Leu Asp Leu Pro Phe Val Thr
    130                 135                 140

Leu Arg Ala Gln Ser Thr Gly Gln His Phe Arg Leu Phe Ile Arg His
145                 150                 155                 160

Gly Pro Leu Gln Val Thr Ala Glu Glu Gly Gly Phe Thr Cys Tyr Gly
                165                 170                 175

Leu Ser Lys Gly Gly Phe Val Pro Trp Phe
            180                 185

<210>  5
<211>  37
<212>  DNA
<213>  Artificial Sequence

<220>
<223>  His aptamer

<400>  5
gggtatattg gcgccttcgt ggaatgtcag tgcctgg                                37


<210>  6
<211>  36
<212>  DNA
<213>  Artificial Sequence

<220>
<223>  TAT aptamer

<400>  6
tgcttcgaac tagggcaaac ggccagctag cgaagc                                 36


<210>  7
<211>  1368
<212>  PRT
<213>  Streptococcus pyogenes

<400>  7